# EUROPEAN PATENT APPLICATION

(11) **EP 1 767 621 A1**
(43) Date of publication of application: **28.03.2007**
(21) Application number: 05751375.6
(22) Date of filing: 08.06.2005
(51) Int. Cl.: C12N 15/00, G01N 33/50, G01N 33/15, C12Q 1/04, C07H 21/04

(54) **METHOD OF PURIFYING ENVIRONMENTAL DNA AND METHOD OF EFFICIENTLY SCREENING PROTEIN-ENCODING GENE FROM ENVIRONMENTAL DNA**

(30) Priority: 08.06.2004 JP 2004170161
(71) Applicant: TOAGOSEI CO., LTD., Tokyo 105-8419 (JP)
(72) Inventor: OKAMOTO, Masaji, c/o Toagosei Co., Ltd., Zsukuba-shi, Ibaraki 3002611 (JP); TANAKA, Kenichi, c/o Toagosei Co., Ltd., Tsukuba-shi, Ibaraki, 3002611 (JP)
(74) Representative: Denison, Christopher Marcus
(86) International application number: PCT/JP2005/010907
(87) International publication number: WO 2005/121336

(57) **Abstract**

The method of purifying environmental DNA and the method of screening for a gene encoding a functional protein from environmental DNA are provided. DNA from a environmental sample is purified by recovering DNA from an environmental sample and allowing the thus recovered DNA to be subjected to gel filtration chromatography and ion exchange chromatography. In addition, a display library of DNA derived from an environmental sample is created so that screening is carried out in terms of the interaction of the above display library with ligands. Thus, screening of protein-encoding genes can be carried out.

## Description

### Technical Field

The present invention relates to a method of purifying DNA derived from an environmental sample (hereafter referred to as "environmental DNA") and a method of efficiently screening for protein-encoding genes from environmental DNA.

### Background Art

### <DNA purification from soil>

Hitherto, in general, DNA used in genetic engineering has been prepared by extraction and purification of pure-cultured microorganisms or cells. Upon preparation, all impurities are substances derived from media and microorganisms used, for example. Recently, methods of purifying DNA from such impurities have been established for various cases.

The term "environment" used herein refers to an environment in which microorganisms live. Examples of such environment include soil, sludge, sewage, rivers, lakes, seawater, seabeds, the insides and the surfaces of tissues of insects, animals, or plants, and excrement.

The term "environmental sample" refers to a sample collected from such an environment. That is, unlike the case of pure culture of microorganisms and cells, for example, it is necessary to isolate DNA from a variety of impurities derived from an environmental sample when DNA is extracted and purified from an environmental sample such as soil or sludge. In particular, soil contains a large quantity of a substance called humin. Humin is a naturally occurring organic substance obtained through polymerization of a degradation product produced from remains of animals and plants subjected to microbial degradation. Humin does not have a specific molecular structure, which every general chemical substance has. The elemental composition of humin is carbon (about 58%), hydrogen (about 4%), and nitrogen (1.5% to 6.0%), for example, and the substantial rest is oxygen. Further, humin includes ash, or the like (2% or less). Humin is classified into humic acid, fulvic acid, and hymatomelanic acid, each of which is alkaline soluble. When humin is extracted using an alkaline solution, and then acidified, the soluble component obtained at pH of 1 is fulvic acid. The precipitate is subjected to alcohol extraction such that the alcohol-soluble component, which is hymatomelanic acid, is obtained. The insoluble component is humic acid. In addition, plant-derived humic acid occasionally refers to humus acid. Further, humic acid can also be derived from animal planktons in oceans and lakes.

Humic acid has molecular weight distribution and electric charge similar to those of DNA. Thus, when DNA is purified from an environmental sample, humic acid behaves like DNA, which is a significant obstacle. The degree of DNA purification can be simply determined based on ultraviolet-visible absorbance waves and ratios of absorbance at λ 260 nm to that at λ280 nm and that at λ260 nm to that at λ230 nm. The DNA, the protein, and the humic acid have maximum absorbances at λ260 nm, λ280 nm, and λ230 nm, respectively. When the sample contains a protein or humic acid in an amount larger than that of DNA, the ratios of absorbance at λ 260 nm to that at λ280 nm and that at λ260 nm to that at λ230 nm decrease. The purity of DNA can be selected according to the purposes of use. In general, in the case of high-purity DNA, the ratio of absorbance at λ260 nm to that at λ280 nm equals to 1.8 to 2.0 and the ratio of absorbance at λ260 nm to that at λ230 nm exceeds 2.0.

Hitherto, there have been several reports on methods of purifying DNA derived from a soil sample (hereafter referred to as "soil DNA"). Table 1 lists those reports with the purification protocols used therein.

**Table 1**

| <Protocol comparison> | | | | | | | | |
|---|---|---|---|---|---|---|---|---|
| Reference | Buffer | Disruption | Extraction | Purification | Yield | Purity | | Results |
| | | | | | µg/g(soil) | A260/A280 | A260/A230 | |
| Non-Patent Document 1 | 0.25 M TH8.0, 0.15 M NaCl, 100 mM EDTA (pH 8.0), 1% CTAB | 15 mg/ml lysozyme, 37°C, 1h, 4 × FT | 5% SDS, TH-sat phenol, P/C ext., Isop-OH ppt. RNaseA | Elutip-d (S&S, ion-exchange) | 10 (Mt) | 1.1 | 1.1 | Cannot be digested by restriction enzymes |
| | | | | | 200 (farm) | 1.2 | 0.8 | |
| Non-Patent Document 2 | 0.1 M PB8.0 | Bead beating, 3300 rpm, 2 min. | 50% P/C, 5% SDS | Sephadex G-200 | 8 (farm) 31 (forest) | Not presented | Not presented | Worked for PCR |
| Non-Patent Document 3 | 100 mM TH8.0, 25 mM NaCl, 2.5% SDS, 25% Cloroform | Bead beating | SDS/chloroform | Sepharose 2B | Not presented | Not presented | Not presented | Worked for PCR |
| Non-Patent Document 4 | 0.225 M NaCl, 150 mM EDTA (pH 8.5) | 17 mg/ml lysozyme, 37°C, 30 min. | 0.5% SDS, 5 mg/ml ProK, pH 8.5, 37°C, 1h | 1% CTAB ppt | Not presented | Not presented | Not presented | Worked for PCR |
| Non-Patent Document 5 | 0.2MPB8.0, 0.1 M NaCl, 50 mM EDTA (pH 8.0), 1 mM DTT, 0.2% CTAB | Bead beating | Phenol/CHCl3/isoamyl-OH, CHCl3/isoamyl-OH | PEG6000 | 30-300 (cray, loam) | Not presented | Not presented | Worked for PCR |
| Non-Patent Document 6 | 250 mM TH8.0, 125 mM NaCl, 50 mM EDTA | 5 x Freeze-thaw, ultrasonic | 1% SDS, proteinase K, phenol/CHCl3 | ULTRAFREE C3 PROBIND | Not presented | Not presented | Not presented | Worked for PCR |
| Non-Patent Document 7 | 120 mM K2HPO4, 0.7 M NaCl, 5% CTAB, 50% P/C | Bead beating | Phenol/CHCl3/isoamyl-OH, CHCl3/isoamyl-OH, isopro-OH | Sepharose CL-4B | 7 | 1.15 | 1.42 | Worked for PCR |
| Non-Patent Document 7 | Tris-HCl | Bead beating | SDS, GTC, G-HCl | UltraClean | 4 | 1.02 | 1.31 | 1 x worked for PCR |
| *Standard pure DNA | | | | | | 1.8-1.9 | >2.0 | |

As shown in table 1, in accordance with conventional methods of purifying soil DNA, the highest ratios of absorbance at λ 260 nm to that at λ280 nm and that at λ260 nm to that at λ230 nm of purified soil DNA are approximately 1.2 and 1.4, respectively. Thus, the purity of such DNA is low. In addition, Non-Patent Document 3 listed in table 1 teaches that sepharose 2B is the most useful example based on examination of effects of separation between DNA and humic acid depending on types of gel filtration carriers.

Meanwhile, Patent Document 1 discloses that soil is subjected to ultrasonication using a buffer containing Tris-HCl, EDTA, NaCl, and polyvinyl pyrrolidone for DNA extraction, that the obtained extract is dissolved in a TE buffer, and that soil DNA is recovered using 4 purification protocols. In the document, the purity was evaluated by allowing purified soil DNA to be subjected to PCR. In the cases of protocols A (using an Elutip d column (anion exchange resin) alone) and B (using Sephacrl S200 (gel filtration) and an Elutip d column in that order) among the four protocols, soil DNA could not be recovered or PCR products were not detected during PCR. On the other hand, in the case of protocol D (using a Microspin Sephacryl S400 HR column and an Elutip d column in that order), PCR products were detected during PCR.

As described above, the purity of soil DNA purified by conventional soil DNA purification methods is at a level that indicates that such DNA can be used, at best, as a template for PCR; that is to say, a sufficient level of purity has not been achieved.

In recent years, it has been recognized that microorganisms that can be cultured in laboratories accounts for 1% of all microorganisms existing in the environment, including in soil (Non-Patent Document 8). If a DNA library is created by directly recovering DNA from environmental samples without the need to culture microorganisms, it becomes possible to access genes that have been unexamined. Thus, it can be expected that opportunities for cloning useful genes will significantly increase. Therefore, it is desired to provide a method of purifying high-purity environmental DNA that can be used for the creation of a library of interest, which can be enriched.

### <Conventional enzyme screening>

A variety of enzymes that have been found in microorganisms by conventional enzyme screening have been used in various fields in industries related to food processing, chemical products, medicine, agrichemicals, biomass, and the like. Moreover, green chemistry has been proposed for the future against a backdrop of social needs to reduce environmental burdens. Thus, the use of more biocatalysts (i.e., enzymes) in industrial processes has been discussed (Non-Patent Document 9).

Hitherto, many enzymes that have been used industrially were isolated and identified by methods of screening for microorganisms from soil that have desired catalytic activities. A process common among such screening methods is the pure culture of microorganisms. Individual microorganisms are cultured in various types of media under various conditions, followed by random selection of colonies. Alternatively, after being subjected to culture, microorganisms are screened for in relation to antibiotics, nutritional requirement, and the like so that intended miroorganisms can be selected. Further, based on the condition that enzymes of interest are essential for the growth of microorganisms, microorganisms having enzymes of interest can be selected. For instance, thermostable enzymes can be obtained by exclusively screening for microorganisms that can live in an environment of almost 100°C. Meanwhile, enzymes having high activities at a low temperature can be obtained by screening for microorganisms that can actively proliferate at low temperatures. Further, enzymes that are capable of degrading or converting specific target substrates can be obtained by carrying out screening using a medium with which substrate degradation can be recognized based on the presence of a halo or a color reaction. Alternatively, in such case, microorganisms are screened for, such microorganisms proliferating in a medium containing a single carbon or nitrogen source that is a specific target substrate.

### <Drawbacks of conventional enzyme screening>

As described above, in general, conventional enzyme screening methods sometimes require as long as 3 weeks until several to tens of thousands of colonies have been assayed during the step of primary screening of microorganisms so that growth of microorganisms is confirmed. Then, activities of enzymes of interest are examined, such enzymes being found in microorganisms that have been obtained during the step of primary screening. Thus, microorganisms having enzymes exhibiting sufficient levels of abilities are selected. Further, in order to isolate enzyme genes, it is necessary to isolate enzyme genes from selected microorganisms and allow them to be subjected to cloning. Thus, conventional enzyme screening methods are significantly labor- and time-consuming, which have been problematic.

Furthermore, as described above, since it is said that microorganisms that can be cultured in laboratories account for 1% of all microorganisms existing in an environment such as soil, such environment contains many microorganisms that cannot be cultured.

### <Metagenome library>

Thus, in recent years, instead of enzyme screening with the use of culturable microorganisms, an approach to accessing gene resources of interest has been taken, in which an environmental sample containing microorganisms is used without the need to culture microorganisms (Patent Documents 2-3 and Non-Patent Documents 10-12). The outline of such approach is described as follows: DNA is purified from an environmental sample such as soil; purified DNA is ligated to an adequate vector; a library is created by introducing the vector into an adequate host; and a clone having a gene of interest is searched for in the created library. Such library created by recovering DNA from an environmental sample differs from a general library derived from a single type of an organism. Such library consists of a mixture of genomes derived from many types of organisms. The abundance of microorganisms in an environment varies depending on the type of environment. For instance, the abundance in surface soil is high so that up to 10⁸ to 10¹⁰ microorganisms exist in 1 g of surface soil (Non-Patent Document 13). Further, the abundances of individual microorganisms are different; however, at least several to tens of thousands of various types of microorganisms exist together in an environment (Non-Patent Document 14). Thus, a library created from a given environmental sample has an extremely diversified and highly complex configuration. Such library is sometimes referred to as a metagenome library (Non-Patent Document 15).

Herein, important parameters for a conventional gene library are the number of genes or the genome size of a biological species of interest and the library size required for the library to contain 100% of the number of genes or the genome size. For instance, a gene library is created for the purpose of cloning an amylase gene with the use of a useful amylase-producing strain of the genus Bacillus. When considering that the genome size of the strain is 5 Mbp and an amylase gene might be cleaved, a gene library used for amylase gene isolation may be created in a manner such that the gene library has total inserts of 50 Mbp (about 10 times the 5-Kbp average insert of a single clone). In other words, such gene library contains 10⁴ independent clones. As described above, when it is obvious that a gene of interest exists among genes contained in a single biological species, the number of clones that should be contained in a gene library can be determined.

### <Drawbacks of conventional metagenome libraries>

Meanwhile, a metagenome library is a highly complex mixed-genome library containing genes of several thousands of types of microorganisms or more. Thus, when a gene of interest is isolated from a metagenome library, a tremendous number of clones must be screened for. As described above, it can be expected that a gene having a function that has been unknown can be isolated using a metagenome library. On the other hand, it is important that high throughput screening is carried out when conducting such screening.

In the cases of existing metagenome libraries, vectors used are cosmid vectors or BAC vectors that can contain large DNA fragments having sizes of 20 to 100 Kbp. When a vector that can contain a large DNA fragment is used, a gene of interest can easily be recovered intact. However, DNA in a metagenome library is derived from an unknown microorganism. It has been well-known that the mechanism involved in gene expression regulation varies depending on biological species. For instance, the Shine-Dalgarno sequence located upstream of an initiation codon is complementary to the 16S ribosome RNA sequence. The distance between the Shine-Dalgarno sequence and the initiation codon influences translation initiation frequency. In addition, the Shine-Dalgarno sequence located upstream in many Escherichia coli genes is not recognized in Bacillus subtilis. Thus, an Escherichia coli gene is not translated when being introduced into Bacillus subtilis (Non-Patent Document 16). As described above, a heterologous gene is not always expressed in other hosts. Thus, when a means of screening a metagenome library is based on the presence of expression proteins, gene expression occurs only in the case of a gene in a host in which transcription is induced and a translation initiation signal functions.

### <Phage display library method and in vitro expression method>

Meanwhile, a phage display library method comprises the following steps. First, peptides of an arbitrary amino acid sequence that has fused with a coat protein are displayed on the surfaces of phages. Then, a group of clones having sequences that bind with affinity to ligand proteins is selected from among phage population having peptides displayed thereon via affinity selection, followed by amplification. This cycle of selection and amplification is repeated such that a clone of interest is enriched (resulting in an improved S/N ratio). In addition, in accordance with the phage display library method, there is a relationship between phages having peptides displayed thereon and peptide-encoding genes. Further, since phages can be amplified, it is possible to search as many as 10¹⁰ to 10¹² clones during a single operation. There have been a variety of applications of the phage display library since it was released approximately 20 years ago (Non-Patent Document 17). For instance, with the use of a random peptide library, identification of an epitope of an antibody or searches for an oligopeptide having novel bioactivity have been carried out. In addition, with the use of an antibody library, a monoclonal antibody can be obtained in a short period of time. Further, with the use of an antibody library, an antibody having improved high affinity has been produced. Also, it has been attempted to obtain an enzyme from an enzyme variant library. Further, as an example of combinatorial library technology whereby clones can be amplified, a phage display library is also employed in a method based on expression in an in vitro transcription and translation system that is excellent in terms of diversity as represented by ribosome display (Non-Patent Document 18).
Patent Document 1: JP Patent Publication (Kohyo) No. 2003-520578 A
Patent Document 2: JP Patent Publication (Kohyo) No. 2000-513933 A
Patent Document 3: JP Patent Publication (Kohyo) No. 2001 - 520055 A
Non-Patent Document 1: C. Joe-Chan et al., J. Microbiol., 34(3): 229, 1996
Non-Patent Document 2: D. N. Miller et al., Appl. Environ. Microbiol., 65 (11): 4715, 1999
Non-Patent Document 3: D. N. Miller, J, Microbiological Methods, 44, 49, 2000
Non-Patent Document 4: C. Schabereiter-Gurtner et al., J. Microbiol. Methods, 45: 77, 2001
Non-Patent Document 5: H. Burgmann et al., J. Microbiol. Methods, 45: 7, 2001
Non-Patent Document 6: D. A. Santosa, Molecular Biotechnology, 17: 59, 2001
Non-Patent Document 7: E. M. James et al., FEMS Microbiol. Ecol., 36: 139, 2001
Non-Patent Document 8: Amann RI, Ludwig W, Schleifer KH., Microbiol Rev., 59(1): 143-69, 1995
Non-Patent Document 9: Hiromichi Ohta, "Biocatalysts and green chemistry," Chemical Engineering, vol. 46, no. 7, pp. 49-53, 2001
Non-Patent Document 10: Rondon, M. R. et al., Appl. Environ. Microbiol., 66(6): 2541-7, 2000
Non-Patent Document 11: MacNeil, I. A. et al., J. Mol. Microbiol. Biotechnol., 3(2): 301-8,2001
Non-Patent Document 12: Knietsch A. et al., J. Mol. Microbiol. Biotechnol., 5(1): 46-56, 2003
Non-Patent Document 13: Tadayuki Imanaka (eds.), "Great expansion of the use of microorganisms (Biseibutsu riyou no daitenkai)," NTN, pp. 250-254, 2002
Non-Patent Document 14: Torsvik V. et al., Appl. Environ. Microbiol., 56(3): 782-7, 1990
Non-Patent Document 15: Handelsman J, Rondon MR, Brady SF, Clardy J, Goodman RM, Chem. Biol., 5(10): R245-9, 1998
Non-Patent Document 16: Band L, Henner DJ., DNA., 3(1): 17-21, 1984
Non-Patent Document 17: Smith, G. P., Curr. Opin. Biotechnol., 2(5): 668-73, 1991
Non-Patent Document 18: Mattheakis, L. C., Bhatt, R. R., and Dower, W. J., Proc. Natl. Acad. Sci. USA., 91, p. 9022, 1994

### Disclosure of the Invention

As described above, the purity of soil DNA that has been purified by a conventional method of purifying soil DNA has not been sufficient when used in a variety of genetic engineering treatments. Thus, in accordance with the present invention, high-purity environmental DNA is prepared, which is available for reactions with high impurity sensitivity such as phage packaging reactions and reactions in in vitro transcription-translation systems. Meanwhile, genes in metagenome libraries are derived from unknown microorganisms. Thus, it has been unclear whether or not such genes are expressed in hosts used. Therefore, a metagenome library contains genes with low expression efficiency and those which are not expressed, which has been problematic. Further, upon screening of a metagenome library, it is necessary to screen for a tremendous number of clones. It is desired that such screening be carried out with efficiency.

Under the above circumstances, it is an objective of the present invention to provide a method of purifying high-purity environmental DNA by removing impurities from an environmental sample. Further, it is another objective of the present invention to provide a method of efficiently screening for protein-encoding genes, such genes being screened for in a display library created using environmental DNA.

As a result of intensive studies to achieve the above objectives, the inventors of the present invention have found that high-purity environmental DNA derived from an environmental sample can be obtained by recovering DNA from an environmental sample and allowing the DNA to be subjected to gel filtration chromatography and/or ion exchange chromatography. Further, they have found that protein-encoding genes can efficiently be screened for by creating a phage display library or an in vitro expression library of environmental DNA derived from an environmental sample and carrying out screening based on the interaction of the above library with ligands. This has led to the completion of the present invention.

The present invention encompasses the following (1) to (16):
(1) a method of screening for protein-encoding genes, comprising creating a display library of environmental DNA derived from an environmental sample and carrying out screening based on the interaction of the library with ligands;
(2) the method of screening for protein-encoding genes described in (1), characterized in that the environmental DNA derived from an environmental sample is purified by a method of purifying environmental DNA derived from an environmental sample, comprising recovering DNA from an environmental sample and allowing the DNA to be subjected to gel filtration chromatography and/or ion exchange chromatography;
(3) the method of screening for protein-encoding genes described in (2), characterized in that the method of purifying environmental DNA derived from an environmental sample comprises recovering DNA by disrupting the environmental sample using glass beads;
(4) the method of screening for protein-encoding genes described in (2), wherein the method of purifying environmental DNA derived from an environmental sample comprises recovering DNA from an environmental sample and allowing the recovered DNA to be subjected to partial degradation;
(5) the method of screening for protein-encoding genes described in (2), characterized in that the exclusion limit of the gel filtration chromatography is 10⁵ Da or more;
(6) the method of screening for protein-encoding genes described in (2), characterized in that the size of DNA purified by the method of purifying environmental DNA derived from an environmental sample is 0.5 to 5 Kbp;
(7) the method of screening for protein-encoding genes described in (1), characterized in that the ligands are saccharide;
(8) the method of screening for protein-encoding genes described in (7), characterized in that the saccharide is selected from the group consisting of cyclodextrin, amylose, and acarbose;
(9) the method of screening for protein-encoding genes described in (1), comprising the step of immobilizing ligands to immobilization carriers before the screening;
(10) a method of screening for a clone of interest based on the expressed activity of an insert gene, comprising amplifying a DNA fragment enriched by the method of screening for protein-encoding genes described in (1), transferring the amplified fragments to an expression vector, and introducing the vector into a host;
(11) the method of screening for protein-encoding genes described in (1) using a combination of two or more types of ligands and/or carriers;
(12) a method of purifying environmental DNA derived from an environmental sample, comprising recovering DNA from an environmental sample and allowing the DNA to be subjected to gel filtration chromatography and/or ion exchange chromatography;
(13) the method of purifying environmental DNA derived from an environmental sample described in (12), characterized in that DNA is recovered by disrupting an environmental sample using glass beads;
(14) the method of purifying environmental DNA derived from an environmental sample described in (12), comprising recovering DNA from an environmental sample and allowing the recovered DNA to be subjected to partial degradation;
(15) the method of purifying environmental DNA derived from an environmental sample described in (12), characterized in that the exclusion limit of the gel filtration chromatography is 10⁵ Da or more; and
(16) the method of purifying environmental DNA derived from an environmental sample described in (12), characterized in that the size of the purified DNA is 0.5 to 5 Kbp.

Hereafter, the present invention will be described in greater detail.

The method of purifying environmental DNA derived from an environmental sample of the present invention is a method comprising recovering DNA from an environmental sample and allowing DNA to be subjected to gel filtration chromatography and/or ion exchange chromatography. In accordance with the method of purifying environmental DNA of the present invention, high-purity environmental DNA can be obtained.

In the present invention, the term "environment" refers to an environment in which microorganisms live. Examples of such environment include soil, sludge, sewage, rivers, lakes, seawater, seabeds, the insides and the surfaces of tissues of insects, animals, or plants, and excrement.

In the present invention, the term "environmental sample" refers to a sample obtained from such an environment. Further, such sample is preferably collected from soil, sludge, a seabed, or the like. Particularly preferably, it contains humic acid. In addition, it is preferable that such environmental sample be not a sample obtained via culture with the addition of a medium to a sample obtained from an environment.

In the present invention, the term "environmental DNA" refers to DNA obtained from an environmental sample.

In the present invention, the term "soil DNA" refers to DNA obtained from a soil sample as an environmental sample.

In the present invention, the term "display library" refers to a phage display library and an in vitro expression library.

In the present invention, the term "pulverization" includes the fine crushing of the mass of an environmental sample. Such operation indicates extracellular release of DNA in cells of microorganisms or the like in an environmental sample.

In accordance with the method of purifying environmental DNA of the present invention, first, DNA is recovered from an environmental sample. The outline of the method will be described below.

When an environmental sample forms a mass, sufficient disruption takes place. In addition, any means can be applied as long as DNA in microorganisms in an environmental sample can be extracted. For instance, pulverization is a means of extracting DNA. As a means of disruption, bead beating, sonication, and the like can be used.

Then, an environmental sample is suspended in an adequate amount of a buffer (e.g., in an amount equivalent to that of an environmental sample). Examples of such buffer include Tris-HCl and PBS. In addition, the pH of such buffer ranges from neutral to moderate alkaline. Specifically, the pH is preferably pH 7 to 9 and more preferably pH 7 to 8. In addition, the salt concentration of a buffer is preferably 0.1 to 2 M and more preferably 1 to 2 M. Further, it is possible to sufficiently disperse an environmental sample with the addition of surfactants such as TritonX-100, Tween20, or Nonidet P-40 to a buffer.

Subsequently, glass beads are added to the above suspension containing the environmental sample, followed by a pulverization treatment. The amount by weight of glass beads used is preferably 1/3 to 2 that of the environmental sample and more preferably 1/2 of the same. The particle sizes of glass beads added are preferably 50 to 1000 µm and more preferably 100 to 500 µm.

Then, a vessel accommodating the suspension and the glass beads is hermetically sealed, followed by shaking agitation. The material of the beads may be a material other than glass, as long as it is hard material. As a result of the aforementioned operation, it is possible to disrupt the cells of microorganisms contained in an environmental sample. Alternatively, cells of microorganisms or the like may be disrupted via French press, ultrasonication, or the like.

After disruption of cells of micoroorganisms contained in the environmental sample, the supernatant containing environmental DNA is recovered via centrifugation, filtration, or the like. The recovered supernatant is subjected to conventional methods such as phenol treatment and ethanol precipitation, such that proteins contained in the supernatant are removed, resulting in extraction of environmental DNA. Alternatively, when the suspension is subjected to shaking agitation together with glass beads in the above case, the salt concentration of the suspension is adjusted to a high concentration of 1 to 2 M after shaking agitation, such that environmental DNA is allowed to adsorb to glass beads. After adsorption, glass beads to which environmental DNA have adsorbed are washed, followed by DNA elution treatment. As a result, environmental DNA can be recovered. As described above, with the use of glass beads to which environmental DNA can adsorb, environmental DNA can readily be obtained from an environmental sample. Thus, in accordance with the present invention, such glass beads are preferably used.

In accordance with the method of purifying environmental DNA of the present invention, recovered environmental DNA is allowed to react with deoxyribonuclease (hereafter referred to as "DNase") so as to be partially degraded, resulting in the obtaining of the lower molecular weight of environmental DNA. A preferred example of DNase is DNase having a low level of specificity such as type I nuclease. For instance, such DNase may be DNase I. Alternatively, environmental DNA may be subjected to ultrasonication such that environmental. DNA is physically sheared, resulting in the obtaining of lower molecular weight of environmental DNA. In addition, conditions of partial degradation are selected based on the assumption that desired DNA sizes can be obtained after the final purification. Table 2 below lists reaction conditions under which many environmental DNA fragments of 2 Kbp in size can be obtained with the use of DNase I.

**Table 2**

| Reaction condition | |
|---|---|
| Buffer composition: | 30 mM NaOAc, pH 5.2, 10 mM MgCl₂ |
| DNA concentration: | 450 µg/ml |
| DNase I: | 15 U/ml |
| Reaction temperature and time: | 25°C, 15 minutes |

Meanwhile, in accordance with the method of purifying environmental DNA of the present invention, recovered environmental DNA is subjected to gel filtration chromatography. The exclusion limit upon gel filtration chromatography is 10⁵ Da or more and more preferably 5 x 10⁶ Da or more (converted in terms of dextran). Examples of a gel filtration carrier and/or column used in gel filtration chromatography include Sephacryl S-300HR, Sephacryl S-400HR, Sephacryl S-500HR, Sepharose CL4B, Sepharose CL6B, Superose 6, and Superose 12. Further, a carrier and/or column for high performance liquid chromatography (hereafter referred to as "HPLC") may be used as long as the aforementioned exclusion limit is obtained.

Examples of a buffer used in gel filtration chromatography include TE (10 mM Tris-HCl, pH 8.0, 1 mM EDTA) and TES (10 mM Tris-HCl, pH 8.0, 1 mM EDTA, 0.2 M NaCl).

After environmental DNA is subjected to gel filtration chromatography, it is possible to confirm whether or not a fraction obtained by elution contains environmental DNA by allowing a portion of the fraction to be subjected to agarose gel electrophoresis, for example. In addition, the site of purity elution of environmental DNA of an obtained fraction can be determined based on ultraviolet-visible absorbance waves and ratios of absorbance at λ260 nm to that at λ280 nm and that at λ260 nm to that at λ230 nm, following measurement of the absorbance of the fraction. A portion of each fraction may be subjected to agarose gel electrophoresis so as to select a fraction having a desired size of environmental DNA.

In accordance with the method of purifying environmental DNA of the present invention, fractions containing environmental DNA obtained by gel filtration chromatography may be subjected to ion exchange chromatography such that the purity of environmental DNA can be improved. A preferred example of resin used is weak anion exchange resin such as DEAE. For instance, environmental DNA is allowed to adsorb to resin using a buffer having low ionic strength, comprising 50 mM Tris-HCl (pH 8.0), 1 mM EDTA, and 0.2 M NaCl, for example. Impurities except for environmental DNA are washed and removed using the same buffer having a salt concentration of about 0.5 M NaCl. Then, environmental DNA is eluted using the same buffer having a salt concentration of about 1.0 M NaCl.

After ion exchange chromatography, absorption measurement is carried out so that the purity of the obtained environmental DNA is determined based on ultraviolet-visible absorption waves and absorption ratios of absorbance at λ260 nm to that at λ280 nm and that at λ260 nm to that at λ230 nm. When the ratio of absorbance at λ260 nm to that at λ280 nm is 1.8 to 2.0 and/or when the ratio of absorbance at λ260nm to that at λ230mn is 2.0 or more, it can be determined that environmental DNA can be separated from impurities such as humic acid and proteins, and high-purity environmental DNA is obtained.

In accordance with the method of purifying environmental DNA of the present invention, gel filtration chromatography and ion exchange chromatography may be performed in that order or in the inverse order. Also, either one of them may be carried out alone. In accordance with the method of purifying environmental DNA of the present invention, both gel filtration chromatography and ion exchange chromatography are preferably carried out.

When the size of environmental DNA is specified in accordance with the method of purifying environmental DNA of the present invention, DNA fragments are excised after being subjected to agarose gel electrophoresis. Then, the fragments can be purified using a commercially available spin column such as QIA quick (Qiagen).

In accordance with the method of purifying environmental DNA of the present invention, environmental DNA can be purified so as to have a size of 0.5 to 5 kbp, more preferably 0.6 to 4 kbp, and particularly preferably 0.8 to 3 kbp, for example.

As described above, in accordance with the method of purifying environmental DNA of the present invention, high-purity environmental DNA can be obtained. Impurities tend to influence genetic engineering treatments involving the creation of a gene library. Such treatments involve a variety of reactions related to restriction enzyme treatments, PCR, ligation, transformation, packaging into phage particles, and in vitro transcription and translation. In the case of environmental DNA obtained by the method of purifying environmental DNA of the present invention, since such environmental DNA has high purity, it can be used in a variety of the above types of reactions without inconvenience.

Meanwhile, the method of screening for protein-encoding genes of the present invention (hereafter referred to as "method of screening of the present invention") is a method comprising creating a display library of environmental DNA, enriching a group of clones recognizing ligands based on the interaction of the above display library with ligands, and screening for such clones. In accordance with the method of screening of the present invention, genes encoding proteins recognizing ligands in environmental DNA can be batch-searched from a number of clones. Thus, time- and labor-saving screening can be carried out. Herein, the term "protein-encoding genes" refers to genes or gene fragments encoding proteins having activities or functions.

An example of environmental DNA used in the method of screening of the present invention is environmental DNA purified by the method of purifying environmental DNA of the present invention described above.

In accordance with the method of screening of the present invention, a display library of environmental DNA is created at first.

Examples of methods of selecting desired clones from a display library include the phage display method, the ribosome display method (L. C. Matthekis et al., Proc. Natl. Acad. Sci. USA., 91, p. 9022, 1994), the emulsion method (Tawfik D. S. & Griffth, A. D., Nat. Biotechnol., 16, pp. 652-656, 1998), the STABLE method (Doi, N. and Yanagawa, H., FEBS Lett., 457, pp. 227-230, 1999), and the in vitro virus method (Roberts, R. W & Szostak, J. W., Proc. Natl. Acad. Sci. USA., 94, p. 12297).

Upon creation of a display library, environmental DNA is first ligated to a vector. Examples of such vector that can be used include a λ phage, a T7 phage, an M13 phage, and a T4 phage.

Upon insertion of environmental DNA to a vector, environmental DNA is cleaved with an adequate restriction enzyme and the resulting fragment is inserted into a restriction enzyme cleavage site or multi-cloning site of an adequate vector DNA, resulting in ligation between environmental DNA and a vector. Also, after environmental DNA is blunt-ended, it is possible to ligate an adaptor to blunt-ended environmental DNA in a manner such that the environmental DNA can be applied to a restriction enzyme cleavage site or multi-cloning site of a vector.

In addition, in the case of a vector, environmental DNA can be inserted into the inside of a gene encoding a protein that can be expressed in a host or into the nucleotide sequence encoding the N- or C-terminal of such protein. For instance, in the case of a phage display library, environmental DNA is inserted into the inside of a gene encoding a coat protein that exists in a phage or into the nucleotide sequence encoding the N- or C-terminal of such coat protein. Accordingly, a protein or peptide encoded by environmental DNA can be presented as a fusion protein with a coat protein on a phage. Thus, a protein or peptide encoded by environmental DNA is presented on a phage.

Further, a vector can comprise a gene expression regulatory region, a reporter gene, and a selected marker, for example, in addition to environmental DNA. An example of a gene expression regulatory region is a cis element such as a promoter, a translation initiation signal, or an enhancer. Examples of a reporter gene include: fluorescence and luminescence genes such as GFP and luciferase; and genes of proteins serving as antigens of available antibodies. Examples of a selected marker include antibiotic-resistant genes such as Amp^{r}, Te^{r}, Cm^{r}, Km^{r}, and AUR1-C. In addition, a gene expression regulatory region, a reporter gene, a selected marker, or the like is inserted into a vector in a manner similar to that used in the aforementioned method of inserting environmental DNA into a vector.

Then, a transformant can be obtained by introducing a recombinant vector containing environmental DNA into a host. Such host is not particularly limited as long as the recombinant vector can proliferate therein.

A method of introducing the recombinant vector into a bacterium is not particularly limited as long as DNA is introduced into the bacterium by such method. Examples of such method include a method of using phage infection and calcium ions and a method of electroporation.

Subsequently, the obtained transformant is allowed to proliferate such that the recombinant vector containing environmental DNA can be amplified.

In addition, when the vector used as a recombinant vector is phage DNA such as a λ phage, a T7 phage, or a T4 phage, the vector is allowed to infect a host such as Escherichia coli after packaging reaction, resulting in transformation. Then, the transformed Escherichia coli is allowed to proliferate such that recombinant phage DNA can be amplified. Thereafter, the host is subjected to bacteriolysis so that amplified phages can be obtained.

In the case of a phage display library, amplified phages can directly be used as a library. Whether or not phages contain environmental DNA in a phage display library can be confirmed by PCR such as plaque PCR.

In the case of an in vitro expression library, a library can be obtained via translation from template RNA or transcription and translation from template DNA with the use of cell extracts instead of cells. In such case, environmental DNA is ligated to a plasmid vector in a manner such that a protein related to the DNA can be expressed in cell extracts. Alternatively, environmental DNA is ligated to a DNA fragment encoding a fusion protein with which the DNA is fused, and having a promoter, a translation initiation signal, or the like. Accordingly, such environmental DNA can be used as a template.

Meanwhile, in accordance with the method of screening of the present invention, target ligands of a display library of environmental DNA can be immobilized on immobilizing carriers. Alternatively, such target ligands can be labeled.

In accordance with the method of the screening of the present invention, examples of such ligands include: high-molecular-weight enzyme substrates such as polysaccharides, proteins, and lipids; coenzymes such as NADH, NADPH, FMN, SAM, acetyl CoA, ATP, ADP, cAMP, GST, and riboflavin; enzyme inhibitors such as amylase inhibitor and protease inhibitor; low-molecular-weight enzyme substrates such as sugar, amino acids, and fats; in vivo metabolites; and medicines such as pharmaceuticals. Particular examples thereof include saccharides such as cyclodextrin, amylose, and acarbose.

For instance, upon screening of enzymes or enzyme fragments from a display library of environmental DNA, substrates, coenzymes, and inhibitors are used as ligands. In addition, upon screening of highly active hydrolase, screening is carried out on the condition that the binding between enzymes or enzyme fragments and ligands occur without ligand degradation. Further, when insoluble particles such as starch or cellulose are used as ligands, such ligands themselves can be used as a solid phase.

With a combination of two or more types of ligand beads and carriers, a population recognizing both types of ligands or a population recognizing either type of ligand can be selected. Also, a population that directly binds to carriers can be removed.

Examples of immobilization carriers include beads such as agarose beads, immunobeads, hydrophilic beads and magnetic beads and plates such as enzyme immunoassay (EIA) plates. Alternatively, commercially available agarose beads (produced by Sigma-Aldrich, for example) having various types of substances supported thereon can be used as they were. In such case, substances supported on agarose beads serve as ligands.

In accordance with the method of screening of the present invention, target ligands are allowed to interact with immobilization carriers such that the target ligands bind to the immobilization carriers. For instance, with the use of functional groups such as amino, carboxyl, hydroxyl, epoxy, and tosyl that are located on the surfaces of immobilization carriers, ligands can covalently bind to the carriers. When functional groups of immobilization carriers are carboxyl groups, covalent bonds between amino groups and functional groups that exist on ligands to be immobilized (that is to say, amine couplings) can be formed. In addition, in a case where functional groups of immobilization carriers are carboxyl groups, covalent bonds between free thiol groups and reaction groups that exist on ligands to be immobilized (that is to say, ligand thiol couplings) can be formed when carboxyl groups are modified with PDEA. Further, when ligands to be immobilized have carboxyl groups, the ligands are previously allowed to react with PDEA (2-(2-pyridinyldithio) ethaneamine hydrochloride) such that carboxyl groups are modified with PDEA. Furthermore, carboxyl groups on immobilization carriers are activated and then the carboxyl groups are allowed to react with cystamine dihydrochloride. Thereafter, the resultant is reduced with dithiothreitol (DTT) so as to be converted to thiol groups. Then, covalent bonds (disulfide bonds) between thiol groups on the immobilization carriers and carboxyl groups that are modified with PDEA can be formed; that is to say, surface thiol couplings can be formed.

Also, in accordance with the method of screening of the present invention, target ligands are labeled such that clones binding to the ligands can be eluted based on the labels. For instance, labeling with the use of biotinylation or fluorescent material is carried out. For instance, target ligands are subjected to biotinylation such that ligands and a display library are allowed to interact with each other in a solution. Then, streptavidin beads are used such that clones binding to the ligands can be eluted.

In accordance with the method of screening of the present invention, screening can be carried out based on interaction between a display library and ligands. Herein, the term "interaction" refers to affinity binding between a display library and ligands or an enzyme reaction, for example. The screening is carried out via affinity selection. The affinity selection comprises the steps of binding reaction, washing, elution, and the like.

In order to reduce noise as a result of hydrophobic interaction as with the case of EIA, nonpolar surfactants such as approximately 0.05% to 1% Tween 20, Triton-X 100, and NP-40 can be added to a buffer. A general biochemical buffer having a pH ranging from 2 to 12 may be selected depending on the purposes of use. For instance, even if such buffer has a pH level that does not allow phages in a phage display library to live, a single cycle of affinity selection is possible.

Meanwhile, immobilization carriers having ligands supported thereon are kept in a protein blocking agent such as approximately 0.1% to 5% BSA, skim milk, gelatin, and collagen for 1 hour or more so as to be subjected to blocking according to need. In some cases of the use of low-molecular-weight ligands, it is preferable that blocking not be carried out.

A binding reaction during affinity selection can be carried out at -10°C to 80°C according to the intended use. In addition, the reaction time of the binding reaction may be 1 minute to 24 hours depending on the intended binding properties and Kon values. In general, a phage display library is infected with hosts such as Escherichia coli so as to be operated using a lysate containing the hosts. When the lysate contains a substance that inhibits a binding reaction with ligands, the inhibitor is removed via a gel filtration membrane having an adequate pore size or dialysis, followed by substitution of a buffer. Then, the resultant can be used. Alternatively, phages in the lysate are allowed to precipitate with polyethylene glycol or the like, followed by supernatant exchange. Then, the resultant may be used.

During washing upon affinity selection, immobilization carriers having ligands supported thereon are mixed with a washing buffer in a volume 10 to 50 times that of the carriers for 1 minute or more, followed by buffer exchange. Such buffer exchange is repeated 1 to 20 times, for example. The washing buffer is removed via suction after centrifugation or via centrifugal filtration of immobilization carriers having ligands supported thereon. Alternatively, the washing buffer may be removed by a filter unit and a spin column, or by a vacuum system. In addition, washing can be carried out using a two-phase partition method or magnetic beads.

Meanwhile, in general, elution during affinity selection is preferably performed in a competitive manner using a ligand solution that is competitive to target ligands under moderate conditions. Also, elution can be performed under strong conditions such as low or high pHs and low or high temperatures at which interaction between target ligands and clones in a display library are disturbed, or using a buffer containing urea, surfactants, and the like according to need. When an eluent causes inconvenience to a host in subsequent operations, it is possible not to perform elution so as to add the ligands while binding to beads to a host bacterium.

In general, the obtained eluent is subjected to clone amplification. Moreover, additional affinity selection may be carried out using different ligands such that selectivity can be improved.

Phage clones in the eluent obtained from a phage display library can be amplified in a manner such that they are allowed to infect hosts such as Escherichia coli. In addition, when the eluent contains target ligands or a solution that is competitive to target ligands is used, such eluent or solution inhibits amplification cycles. Thus, it can be removed by allowing hosts infected with phages to be subjected to centrifugation following infection of the hosts with phage clones. Alternatively, hosts infected with phages are subjected to bacteriolysis, ligands, or a solution that is competitive to the ligands can be removed from the lysate by buffer substitution. Amplification of phage clones can be confirmed by monitoring a phage titer of the lysate.

Meanwhile, when phages are not used for a display library, gene amplification is carried out by PCR or the like.

After amplification of phage clones or gene amplification by PCR or the like, PCR such as plaque PCR is carried out using phage clones or products obtained by PCR or the like as templates such that environmental DNA subjected to screening is amplified. Thereafter, the amplified product is subjected to agarose gel electrophoresis so as to confirm the environmental DNA. Further, the nucleotide sequence of the environmental DNA can be determined based on sequencing analysis of the environmental DNA.

In accordance with the method of screening of the present invention, when a phage display library is used as a display library, a protein or peptide expressed by environmental DNA is presented as a fusion protein on the surface of a phage. The obtained environmental DNA may not have a complete coding sequence, so that it occasionally lacks a portion of the N- or C-terminal sequence, in general. Thus, in order to obtain a complete coding region based on the sequence information of environmental DNA of the obtained phage clone, it is necessary to compensate for the upstream or downstream coding region in a manner similar to that used for RACE (Rapid Amplification of cDNA ends). A phage display library before screening contains clones that have not fused because it has an untranslated region. Therefore, in order to obtain the sequence information of the upstream or downstream coding region, PCR is carried out based on the sequence information obtained from a clone using an outward primer, a primer comprising an adaptor sequence or a phage DNA sequence, and a phage display library DNA before screening as a template.

After the upstream or downstream coding region is obtained as described above, ligation is performed by, for example, splicing or recombinant PCR (Higuchi, R., "Recombinant PCR," PCR Protocols, pp. 177-183, M. A. Innis et al., Academic Press, Inc., 1990) such that a complete coding region can be obtained.

In addition, the sequence information of environmental DNA of the obtained phage clone is subjected to matching with a gene database at the nucleotide sequence or amino acid sequence level, followed by homology analysis. Accordingly, the function or activity of a protein encoded by the environmental DNA can be predicted.

When a gene or gene fragment is isolated from the environmental DNA so as to be identified, the gene or gene fragment is introduced into a host as described above such that a transformant can be obtained. In such transformant, a protein or peptide encoded by the gene or gene fragment is allowed to be expressed such that the function or activity of the protein or peptide can be examined.

Enriched population may contain clones that maintain a region that is capable of exhibiting catalytic activity, such region not being a complete coding region. In addition, in the case of the use of labor-saving techniques, inserts of enriched population are amplified together by PCR or the like using primers of consensus sequences such as adaptor or vector sequences. Then, the amplified product is ligated to an adequate intracellular expression vector or secretory expression vector such that a group of transformants can be obtained, which can constitute an enriched expression mini-library. Accordingly, active clones can be selected using an agar medium containing a reagent which can be visually observed for selection as used in conventional methods. The method of the present invention differs from conventional methods in that the population of clones that recognize and bind to a specific ligand is enriched approximately 1000-fold. Thus, the improved frequency of detection of clones of interest can be expected.

A method for activity detection can be carried out as follows. For instance, when hydrolytic activity of a sugar, protein, or fat is detected, water-insoluble substrates such as starch, casein, and fats and oils are mixed with a medium, followed by culture. Then, a degenerated clear zone or halo is searched for. In addition, it is possible to use pH indicators, a variety of reagents for color reaction, fluorescent reaction, and luminescent reaction used for enzyme activity measurement, and antibodies, which are used in conventional enzyme screening.

In accordance with the method of screening of the present invention as described above, screening of protein-encoding genes of interest can be carried out with efficiency using a display library created based on environmental DNA. In accordance with the method of screening of the present invention, when a phage display library is employed, a group of phage clones bound to ligands is concentrated on so as to amplify the same. Thus, selection of protein-encoding genes of interest can be carried out in a very labor-and time-saving manner.

The present specification includes the contents as disclosed in the specification and/or drawings of Japanese Patent Application No. 2004-170161, which is a priority document of the present application.

### Brief Description of the Drawings

Fig. 1A shows an absorbance wave of a pure DNA sample (λ phage) as a positive control that was recovered by UltraClean.
Fig. 1B shows an absorbance wave of soil DNA recovered by UltraClean.
Fig. 2 shows absorbance waves of fractions containing soil DNA after gel filtration chromatography.
Fig. 3 shows agarose gel electrophoresis patterns of soil DNA subjected to partial degradation with DNaseI.
Fig. 4A shows an absorbance at 280 nm of each fraction upon FPLC.
Fig. 4B shows agarose gel electrophoresis patterns of each fraction after FPLC.
Fig. 5 shows absorbance spectral patterns of purified soil DNAs compared with those of purified phage λDNA.
Fig. 6 shows an agarose gel electrophoresis pattern of purified soil DNA.
Fig. 7 shows a scheme of ligation between blunt-ended soil DNA and an adaptor.
Fig. 8 shows an agarose gel electrophoresis pattern of a DNA fragment that was ligated to an adaptor, followed by purification from gel.
Fig. 9 shows agarose gel electrophoresis patterns of library #17.
Fig. 10A shows a transition of the titer of a phage solution in each round.
Fig. 10B shows a transition of the recovery rate based on the number of input phages in each round.
Fig. 11 shows agarose gel electrophoresis patterns of a plaque PCR product in each round.
Fig. 12 shows an alignment of the amino acid sequence of P31 and a homologous sequence in a preserved database.
Fig. 13A shows an alignment of the amino acid sequence of the protein having the highest homology to the amino acid sequence of P31.
Fig. 13B shows an alignment of the amino acid sequence of the protein having the second highest homology to the amino acid sequence of P31.
Fig. 13C shows an alignment of the amino acid sequence of the protein having the third highest homology to the amino acid sequence of P31.
Fig. 13D shows an alignment of the amino acid sequence of the protein having the fourth highest homology to the amino acid sequence of P31.
Fig. 14A shows the bindings of a P31-presenting phage clone to β- and γ-CD-Sepharose 6B beads compared with the case of a wild type phage T7SC1 as a control.
Fig. 14B shows the bindings of a P31-presenting phage clone to β- and γ-CD-Sepharose 6B beads compared with the case of Sepharose 6B beads (S6B) as a control.
Fig. 14C shows inhibition of the bindings of a P31-presenting phage clone to β-and γ-CD-Sepharose 6B beads due to the presence of 1% starch.
Fig. 15 shows an alignment of the amino acid sequence of AE1 and the amino acid sequence of a protein having a homologous sequence.
Fig. 16 shows the nucleotide sequence and the amino acid sequence of P31-encoding gene.
Fig. 17 shows the nucleotide sequence and the amino acid sequence of gene containing insert DNA in AE1.
Fig. 18 shows an alignment of the amino acid sequence of the E.C.3.2.1 family member β-xylosidase, glucosidase and the amino acid sequence of a protein encoded by a single type of a gene fragment obtained in Example 7.
Fig. 19 shows an alignment of the amino acid sequence of the E.C.3.2.1.3, glucoamylase and the amino acid sequence of a protein encoded by a single type of a gene fragment obtained in Example 7.
Fig 20 shows the nucleotide sequence of a single type of gene fragment obtained in Example 7 and the amino acid sequence of a protein encoded by the same.
Fig. 21 shows the nucleotide sequence of a single type of gene fragment obtained in Example 7 and the amino acid sequence of a protein encoded by the same.
Fig. 22 shows an alignment of the amino acid sequence of the E.C.4.2.1.16, dTDP glucose dehydratase and the amino acid sequence of a protein encoded by a gene fragment obtained in Example 8.
Fig. 23 shows the nucleotide sequence of a gene fragment obtained in Example 8 and the amino acid sequence of a protein encoded by the same.

### Best Mode for Carrying Out the Invention

### [Examples]

The present invention is hereafter described in greater detail with reference to the following examples, although the technical scope of the present invention is not limited thereto.

### [Example 1] Purification of environmental DNA

### 1-1. DNA extraction from soil

In a forest located at Ookubo, Tsukuba City, Ibaraki, Japan, black leaf mold was collected from a site 10 cm below surface soil covered with fallen leaves in November 2001. In accordance with the manufacturer's instructions, DNA was extracted from the soil sample (16 g) using UltraClean™ (soil DNA kit Mega Prep (MoBio Lab., Inc.), Catalog #12900-10, Lot #SD1I13) such that approximately 1.4 mg of DNA was obtained. The color of the obtained DNA solution was light brown.

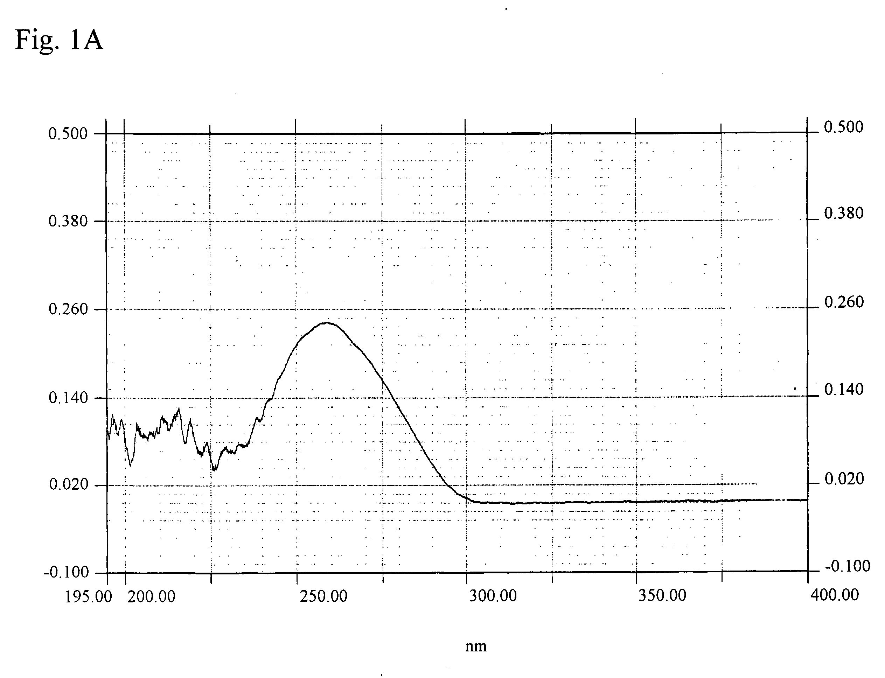

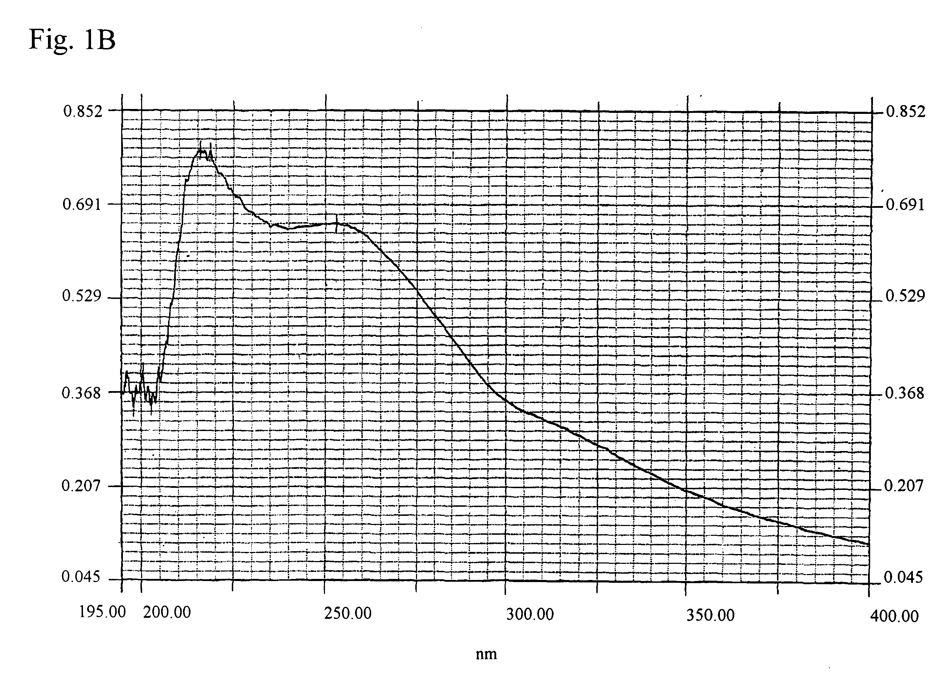

The DNA obtained from the soil (hereafter referred to as "soil DNA") was subjected to absorbance measurement. Figs. 1A and 1B show absorbance waves obtained as a result of the measurement. Fig. 1A shows an absorbance wave of a pure DNA sample (λ phage) serving as a positive control. Meanwhile, fig. 1B shows an absorbance wave of the soil DNA.

In addition, the ratio of absorbance at λ260 nm to that at λ230 nm was 1.49:1 for the pure DNA sample (λ phage) and the same was 0.95:1 for the soil DNA.

### 1-2. Preliminary examination of soil DNA by gel filtration chromatography

Next, the soil DNA solution obtained in 1-1 above was subjected to gel filtration chromatography.

Gel filtration chromatography was performed under the analysis conditions listed in table 3.

**Table 3**

| Analysis condition | |
|---|---|
| Sample: | 270 µl of soil DNA (95 µg) solution |
| Gel: | 3 ml of Sepharose CL4B (Amersham-Pharmacia) |
| Buffer: | TE (10 mM Tris-HCl, pH 8.0, 1 mM EDTA) |
| Fraction volume: | Approximately 350 µl |

During gel filtration chromatography, elution of the soil DNA started in Fr. #4 which is a flow-through fraction, and finished in Fr. #10 or thereabout. Distribution of the brownish substance contained in the soil DNA solution was mainly observed in the low-molecular-weight area.

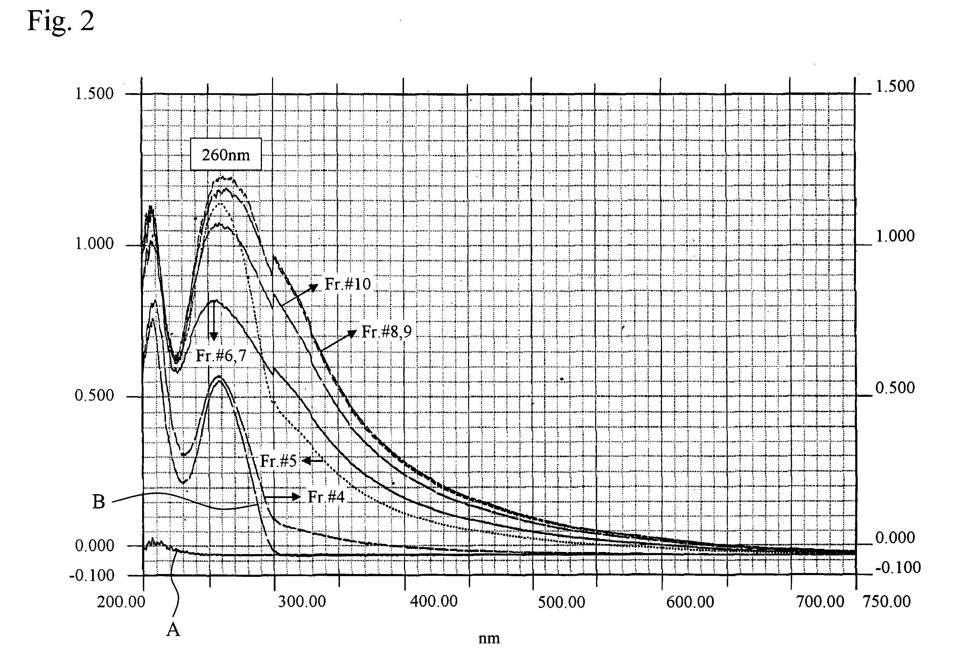

Fig. 2 shows UV absorption waves of the fractions. In fig. 2, continuous line A and broken line B indicate a base line and a line for the pure DNA sample (λ phage DNA), respectively. Further, the ratios of absorbance at λ260 nm to that at λ230 nm and that at λ260 nm to that at λ280 nm of Fr. #4 were 1.83 and 1.57, respectively. Further, the ratios of absorbance at λ 260 nm to that at λ230 nm and that at λ260 nm to that at λ280 nm of Fr. #8 were 2.0 and 1.1, respectively.

Accordingly, since the ratios of absorbance at λ 260 nm to that at λ230 nm were high, gel filtration of the corresponding fraction range was effective for separation of a major part of humic acid. Gel filtration of the corresponding fraction range was effective for the improvement of the degree of purification. However, since the ratios of absorbance at λ 260 nm to that at λ280 nm were low, such gel filtration was found to be insufficient, resulting in the presence of proteins mixed in the fraction.

### 1-3. Examination of conditions of partial degradation of soil DNA

With the use of the soil DNA obtained in 1-1 above, conditions for obtaining the largest amount of 2 Kbp DNA as a result of partial degradation caused by DNaseI were examined. Table 4 lists reaction conditions excluding DNase I. The reaction was terminated with the addition of 5mM EDTA.

**Table 4**

| Reaction condition | |
|---|---|
| Buffer composition: | 30 mM NaOAc, pH 5.2, 10 mM MgCl₂ |
| DNA concentration: | 450 µg/ml |
| Reaction temperature and time: | 25°C, 15 minutes |

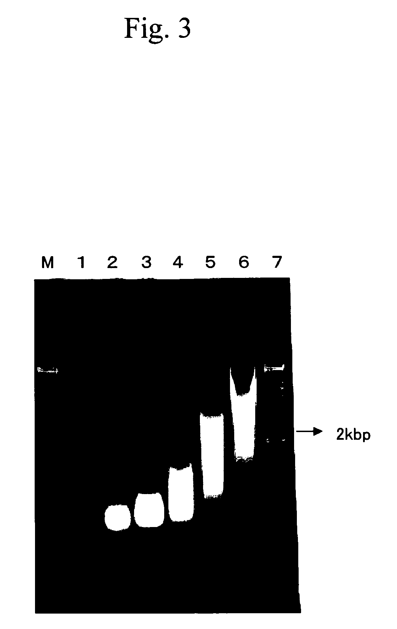

The reaction product was subjected to agarose gel electrophoresis. Fig. 3 shows the results. In fig 3., lanes represent the corresponding samples treated with DNaseI at the following concentrations (U/ml): lane 1: 245 U/ml; lane 2: 122 U/ml; lane 3: 61 U/ml; lane 4: 30.6 U/ml; lane 5: 15.3 U/ml; and lane 6: 0 U/ml. In addition, lanes M and 7 indicate the results of molecular weight markers (1Kb ladder markers).

As is apparent from fig. 3, it was found that the largest number of 2 Kbp DNA fragments were obtained when the soil DNA was treated with DNase I at a concentration of approximately 15 U/ml (i.e., 15.3 U/ml in lane 5).

### 1-4. Purification of soil DNA by FPLC

2 Kbp DNA fragments were obtained by treating the soil DNA obtained in 1-1 above with DNase I (15U/ml) under conditions listed in table 4, followed by partial degradation. Then, for the purpose of removing soil-derived impurities and low-molecular-weight DNA, a sample containing 2 Kbp DNA fragments was subjected to FPLC using a gel filtration column Superose 6 (Amersham-Pharmacia). Herein, FPLC was performed under conditions listed:in table 5 below.

**Table 5**

| FPLC analysis condition | |
|---|---|
| Sample: | 100 µl of a solution containing soil DNA (100 µg) partially degraded by DNase I |
| Gel: | Superose 6 (exclusion limit 4 × 10⁷ Da) φ 10 mm × 300 mm |
| Buffer: | TES (10 mM Tris-HCl, pH 8.0, 1 mM EDTA, 0.2 M NaCl) |
| Flow rate: | 0.3 ml/minute |
| Fraction volume: | Approximately 1 ml |
| Analysis: | Photometric scanning, AGE (Agarose Gel Electrophoresis) |

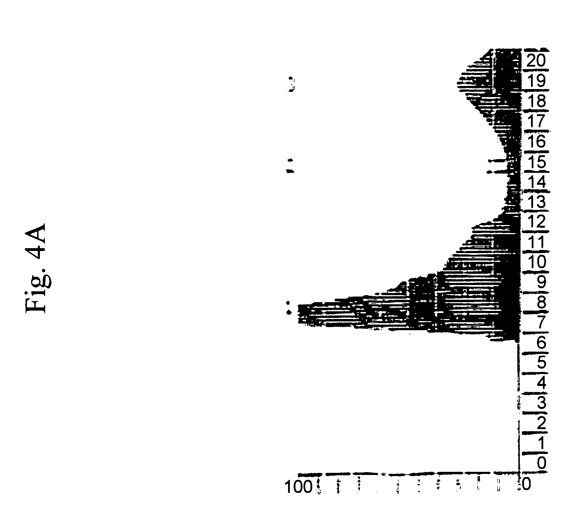

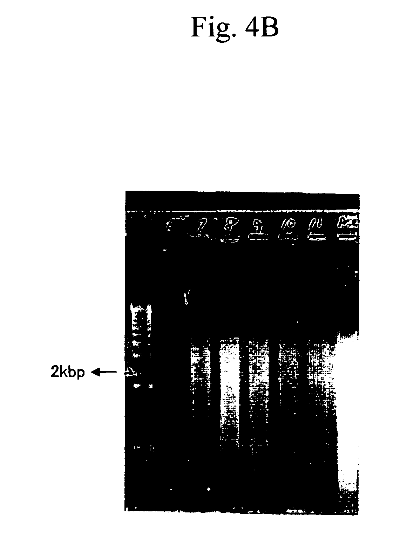

Fig. 4A shows a chart of FPLC (absorbance of each fraction at 280 nm). In addition, fig. 4B shows the agarose gel electrophoresis patterns of Frs. #6 to # 11.

As is apparent from fig. 4A, DNA was eluted after Fr. #7. Further, based on the agarose gel electrophoresis patterns shown in fig. 4B, it was confirmed that absorption after Fr. # 16 was not caused by DNA.

Based on the results of agarose electrophoresis shown in fig. 4B, Frs. #7 to #9 containing few small fragments were recovered such that a DNA pool was obtained. It was intended to exclude DNA fragments having sizes of 0.5 Kbp or less. The absorbance ratios serving as an index of the degree of purification were approximately 1.7 (ratio of absorbance at λ260 nm to that at λ280 nm) and approximately 0.55 (ratio of absorbance at λ260 nm to that at λ230 nm).

### 1-5. Purification by ion exchange chromatography

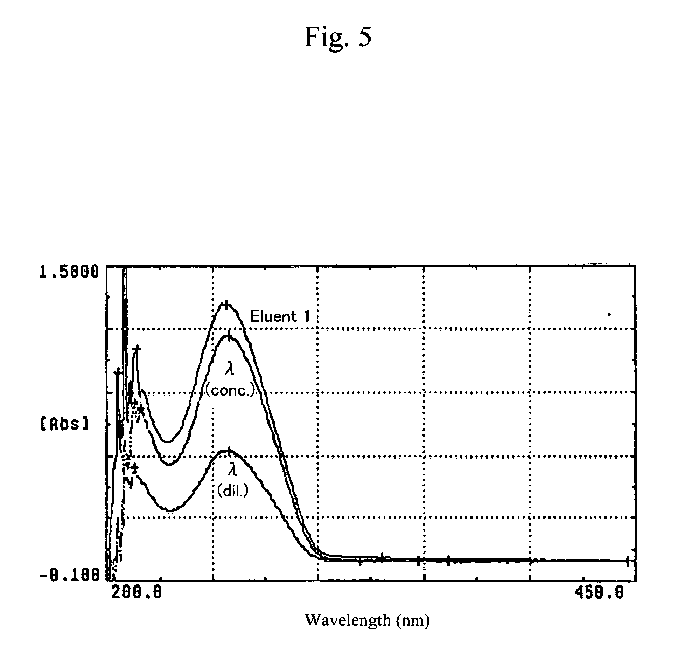

The DNA pool containing Frs. #7 to #9 obtained in 1-4 above (4.5 ml) was purified using a QIAGEN Plasmid Midi kit (100) in accordance with the manufacturer's instructions such that the purity of the DNA pool was improved. Fig. 5 shows spectral patterns of the absorbance of the obtained DNA. In fig. 5, Eluent 1 represents purified DNA of the DNA pool, and λ (conc.) and λ (dil.) represent phage λDNAs of pure DNA samples.

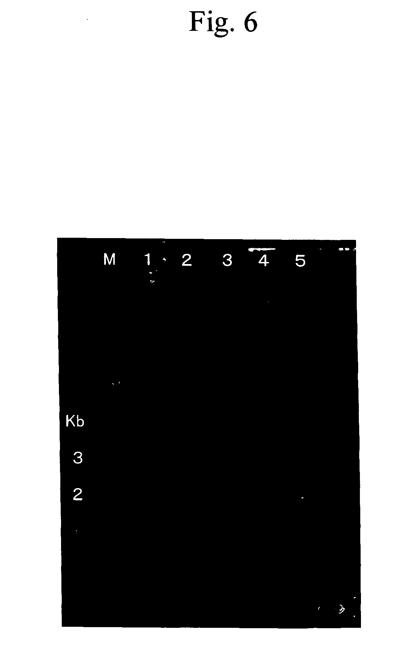

As is apparent from fig. 5, the absorbance spectral pattern of the final purified DNA was favorably equivalent to the same patterns of phage λDNAs that were pure DNA samples. The absorbance ratios of the final purified DNA were 1.91 (ratio of absorbance at λ260 nm to that at λ280 nm) and 2.12 (ratio of absorbance at λ260 nm to that at λ230 nm). Thus, high-purity soil DNA was obtained, compared with that obtained from conventional purified soil DNA. Fig. 6 shows an electrophoresis pattern of purified DNA (lane 4) of the DNA pool. As is apparent from fig. 6, degradation did not occur. The pattern mainly appeared around 2 Kbp.

### [Example 2] Creation of a metagenome display library

### 2-1. Preparation of adaptors

A Hind adaptor was produced by annealing the following synthetic oligonucleotides HdAd5 and pHdAd3 with each other:
HdAd5: 5'-HO-AGCTTAGTGAGTGAGTCCT-3' (SEQ ID NO: 1); and
pHdAd3: 5'-pAGGACTCACTCACTA-3' (SEQ ID NO: 2) ("p" indicates phosphorylation).

Also, an Eco adaptor was produced by annealing the following synthetic oligonucleotides pEcoRIL3 and EcoRIAd5 with each other:
pEcoRIL3: 5'-pGTCGACGCGGCCGCG-3' (SEQ ID NO: 3) ("p" indicates phosphorylation); and
EcoRIAd5: 5'-HO-AATTCGCGGCCGCGTCGAC-3' (SEQ ID NO: 4).

HdAd5 (15 µl) and PHdAd3 (15 µl) (each at a final concentration of 37.5 µM) were mixed together with 20 x SSC (10 µl) so as to produce a Hind adaptor. Meanwhile, pEcoRIL3 (15 µl) and EcoRIAd5 (15 µl) (each at a final concentration of 37.5 µM) were mixed together with 20 x SSC (10 µl) so as to produce an Eco adaptor. Then, these solutions were incubated using a thermal cycler at 96°C for 3 minutes and cooled down to 60°C in an hour. Subsequently, the solutions were incubated at 60°C for 30 minutes and cooled down to 40°C in an hour. Further, the solutions were incubated at 40°C for 30 minutes and immediately cooled down to 25°C, followed by incubation for 10 minutes. Thus, Hind and Eco adaptors were obtained. Annealing of the adaptors was confirmed based on analysis via 5% polyacrylamide gel electrophoresis.

### 2-2. End blunting of soil DNA

The soil DNA purified via ion exchange chromatography in Example 1 was subjected to end blunting using TAKARA Blunting Kits. Subsequently, 2.5 µg of the soil DNA, 1.0 µl of 10 x buffer, and 4.0 µl of distilled water were placed into a 0.5-ml tube. The obtained solution was incubated at 75°C for 5 minutes. T4 DNA polymelase (1.0 µl) was added thereto, followed by gentle stirring using a pipette. Further, the solution was incubated at 37°C for 5 minutes, followed by strong agitation using a vortex mixer, resulting in enzyme deactivation. A TE buffer (40 µl) was added to the solution. Then, the resulting solution was transferred into a 1.5-ml tube.

An equal volume of phenol was added to the solution, followed by extraction twice. An equal volume of chloroform was added thereto, followed by extraction. Thus, 90 µl of a DNA aqueous solution was obtained. Then, glycogen (2.0 µl) and 9.0 µl of 3 M NaOAc were added to the obtained DNA aqueous solution, followed by sufficient agitation. Further, DNA was precipitated with the addition of a 2.5-fold volume of cold ethanol to the DNA solution, followed by centrifugation at 15,000 rpm for 10 minutes. Subsequently, the supernatant thereof was removed. The precipitate was washed with 75% ethanol. After complete removal of ethanol, the precipitate was dissolved in 10 µl of a TE buffer such that a solution containing blunt-ended DNA was obtained.

### 2-3. Ligation of an adaptor to blunt-ended soil DNA

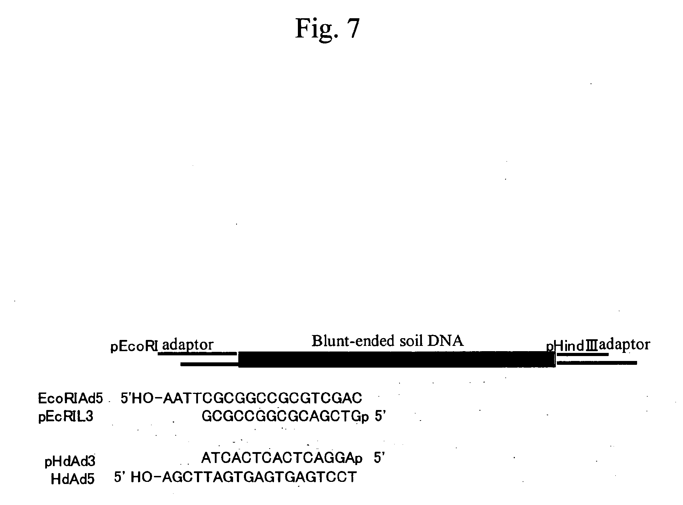

Ligation of an adaptor to blunt-ended soil DNA was carried out using a TAKARA DNA ligation kit Ver. 2. Fig. 7 shows a scheme of ligation of an adaptor to blunt-ended soil DNA. First, 4.4 µl of blunt-ended soil DNA, 11.6 µl of distilled water, and Eco and Hind adaptors (2.0 µl each) in amounts by mole approximately 100 times that of blunt-ended soil DNA were mixed together. An equivalent volume (20 µl) of Solution II and a 2-fold volume (40 µl) of Solution I were added to the mixed solution, followed by incubation at 16°C for 30 minutes. Thus, each adaptor was ligated to blunt-ended soil DNA. The thus obtained DNA solution was subjected to phenol and chloroform extraction, followed by ethanol precipitation. Then, the precipitate was dissolved in a TE buffer.

### 2-4. Purification of ligation products by agarose gel electrophoresis

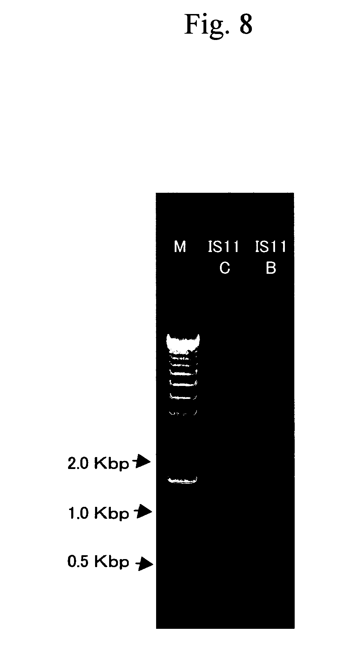

In order to remove unreacted adaptors, the adaptor ligation products obtained in 2-3 above were subjected to agarose gel electrophoresis. Then, 0.5- to 1.3-Kbp and 1.3- to 2.0-Kbp fragments were excised using a molecular weight marker as an index, which had been added to the gel together with the products (fig. 8). In fig. 8, lane M indicates a molecular weight marker and lane IS11C indicates adaptor ligation product IS11C containing 0.5- to 1.3-Kbp fragments. In addition, lane IS11B indicates adaptor ligation product IS11B containing 1.3- to 2.0-Kbp fragments.

The adaptor ligation products that had been excised from gel were subjected to extraction using a Mini Elute Gel Extraction Kit (QIAGEN) in accordance with the manufacturer's instructions. The prepared adaptor ligation products were each inserted into a vector pUC9 Eco/Hind so as to be transformed to Escherichia coli (COMPETENT High DH5α (TOYOBO)). Accordingly, insert DNA derived from soil DNA contained in the adaptor ligation products was evaluated in terms of transformation efficiency. As a result, it was confirmed that the transformation efficiency of each adaptor ligation product (per 100 fmol) was approximately 30% compared with that of a purified plasmid-derived cry 1.5Kb Eco/Hind DNA fragment as positive control DNA.

### 2-5. Coprecipitation of insert DNA derived from soil DNA and vector DNA with ethanol

The adaptor ligation product (hereafter referred to as "insert DNA") and a vector DNA (T7 SELECT 10-3b) were mixed in the composition listed in table 6 below such that a preferred insert/vector ratio was obtained.

**Table 6**

| Library #17 (insert/vector = 4): | |
|---|---|
| Vector DNA solution: | 50 µl (0.5 µg: 21.5 fmol) |
| Insert DNA (adaptor ligation product)IS11C: | 2 µl (86 fmol) |

Subsequently, 2 µl of glycogen and 3 M NaOAc in a volume 1/10 that of the mixed solution were added to the mixed solution, followed by the addition of cold ethanol in an amount 2.5 times that of such solution. The resultant was allowed to stand in ice cold, resulting in DNA precipitation. The precipitate was subjected to centrifugation at 15,000 rpm for 10 minutes so as to be recovered, followed by washing with 75% ethanol. Then, the remaining ethanol was removed therefrom under reduced pressure.

In addition, with regard to library #16 of insert DNA (adaptor ligation product) IS11B, insert DNA (adaptor ligation product) IS11B and vector DNA were subjected to coprecipitation as described above.

### 2-6. Ligation of insert DNA derived from soil DNA to vector DNA

Sterilized distilled water (S.D.W.) (3.0 µl), 0.5 µl of a 10 x buffer, 0.5 µl of 10 mM ATP, and 1.0 µl of T4 DNA ligase were added to the coprecipitate of the above insert DNA and vector DNA such that a reaction solution in a total amount of 5.0 µl was obtained. The reaction solution was incubated in an air incubator at 16°C for 17 hours so that insert DNA and vector DNA underwent ligation reaction. After the reaction, the solution was concentrated to 2.5 µl under reduced pressure so as to be subjected to subsequent packaging operations.

### 2-7. Packaging into T7 phages

The recombinant vector containing insert DNA IS11C obtained in 2-6 above (2.5 µl) was carefully added to 12.5 µl of Packaging extract (Novagen T7 Select 10-3 cloning kit) without causing bubble formation. The resulting solution was incubated at 22°C for 2 hours. After 2 hours, 135 µl of an LB medium was added to the solution, resulting in the termination of the reaction. Thus, a packaging solution in a total amount of 150 µl (hereafter referred to as "library #17") was obtained, which contained T7 phage packaging insert DNA IS11C.

Meanwhile, with the use of insert DNA IS11B, packaging was carried out on a scale twice as large as that used for library #17. Accordingly, a packaging solution (hereafter referred to as "library #16") was obtained, which contained insert DNA IS11B packaged in T7 phages.

In accordance with the manufacturer's instructions of Novagen, a titer of each solution was confirmed via plaque assay. Titers of libraries #16 and #17 were 9.1 x 10⁷ pfu/ml and 2.37 x 10⁸ pfu/ml, respectively.

### 2-8. Insertion frequency

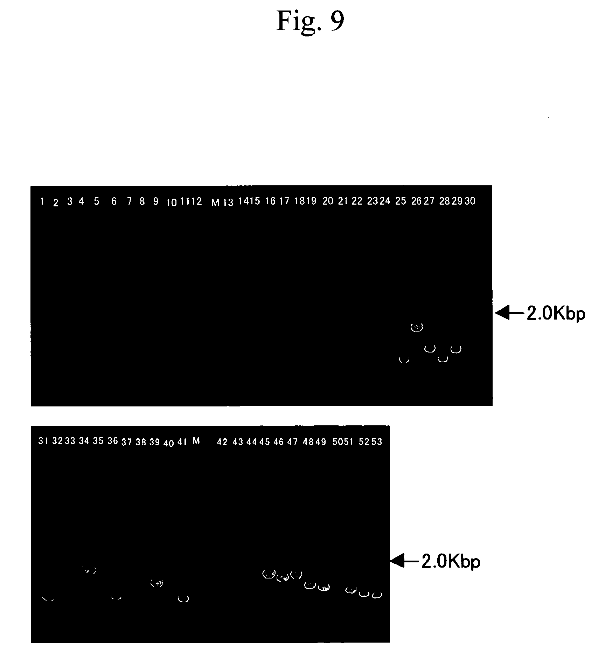

Plaque PCR was conducted so as to examine insertion frequencies of libraries #16 and #17. First, the phage solutions of libraries #16 and #17 were inoculated on separate plates. Plaque formed on each plate was scratched with a toothpick so that it could be suspended in 15 to 20 µl of a PCR reaction mixture (containing primers (0.1 pmol) and Ex Taq enzyme (0.03 U)). At such time, the primers used were T7 up and T7 down primers (Novagen). PCR reaction was carried out using a thermal cycler under the following conditions: 1) 1 cycle at 96°C for 3 minutes; 2) 33 cycles at 94°C for 30 seconds, at 50°C for 30 seconds, and at 72°C for 2 minutes; and 3) 1 cycle at 72°C for 10 minutes. After the termination of the reaction, 1 µl of BPB dye was added to 5 µl of a PCR sample, followed by 0.8% agarose gel electrophoresis at 100 V for 35 minutes. Then, the percentage of phages containing insert DNA was calculated. Fig. 9 shows the results of agarose gel electrophoresis of library #17. In fig. 9, the number of each lane indicates the number of the corresponding PCR sample and lane M indicates a molecular weight marker. The insertion frequency of library #16 was approximately 15%, while on the other hand, the insertion frequency of library #17 was approximately 50%.

### 2-9. Library amplification

Library #16 (300 µl) and library #17 (150 µl) were infected with 5 ml and 10 ml of a host, Escherichia coli BLT5615, respectively, followed by bacteriolysis. Thus, lysate libraries #16P1 and #17P1 were prepared. Both of the titers of lysate libraries #16P1 and #17P1 were approximately 5 × 10¹⁰ pfu/ml. Meanwhile, insertion frequencies were slightly reduced due to amplification treatment. The insertion frequency of library #16P1 was approximately 10%. In addition, the insertion frequency of library #17P1 was approximately 33%.

### 2-10. Analysis of insert DNA nucleotide sequences

Among library #17P1, the nucleotide sequence of a PCR product of each of 20 clones which were confirmed to contain insert DNA by plaque PCR was examined regarding an average of 460 bases. The DNA sequences were subjected to homology search by BLASTn based on an existing nucleotide sequence database such as Genbank (July 2002). The results are shown in table. 7.

**Table 7**

| Homology analysis of insert DNA contained in library #17 | | | | | | | | |
|---|---|---|---|---|---|---|---|---|
| Clone no. | Analyzed base | ORF* (aa) | Blastn** search | Blastx or Tblastx seach** | | Frame | Homology | E-value |
| | | | | Top hit species | Top hit entity | | | |
| 1 | 392 | - | - | - | - | | | |
| 2 | 503 | Δ | - | Methanosarcia acetivorans | Genome | +1 | 36% | 3E-12 |
| 3 | 380 | - | - | Sinorhizobium meliloti | Transposase | -3 | 57% | 3E-42 |
| 4 | 148 | - | - | Streptomyces coelicolor | Genome | -2 | 63% | 2E-34 |
| 5 | 381 | O | - | - | - | | | |
| 6 | 331 | - | - | Clostridium perfringens | Transketorase | -1 | 57% | 1E-21 |
| 7 | 219 | Δ | - | - | - | | | |
| 8 | 262 | - | - | - | - | | | |
| 9 | 479 | Δ | - | Thermoaerobactor tengcongensis | Membrane protein | -3 | 50% | 1E-29 |
| 10 | 277 | Δ | - | - | - | | | |
| 11 | 123 | - | - | Escherichia coli O-157 | Genome | -1 | 40% | 1E-04 |
| 12 | 127 | Δ | - | - | - | | | |
| 13 | 450 | - | - | - | - | | | |
| 14 | 636 | - | - | Clostridium perfringens | Folyl-polyglutamate synthase | -1 | 60% | 1E-27 |
| 15 | 497 | - | - | - | - | | | |
| 16 | 459 | - | - | Rastoniasolanacerum | GSPE-related protein | -3 | 40% | 1E-07 |
| 17 | 204 | O | - | - | - | | | |
| 18 | 145 | - | - | | | | | |
| 19 | 663 | - | - | Mesorizobium loti | mlr5040 protein | -1 | 48% | 2E-58 |
| 20 | 451 | - | - | Burkhoderia mallei | dTDP-4-ketorhamnose reductase | +3 | 47% | 5E-16 |

| | | | | | | | | |
|---|---|---|---|---|---|---|---|---|
| * (-): No ORF in the forward direction; O: In-frame ORF; Δ: ORF with a single-base frameshift ** (-) No significant hit | | | | | | | | |

As shown in table 7, the results of homology analysis do not contain significant hit results, indicating that the nucleotide sequences of all insert DNAs do not have high homology and thus they are novel DNAs. The results support the fact that approximately 99% of the microorganisms in soil have not been identified. Therefore, it has been confirmed that display libraries of genetic resources of such microorganisms can be created.

Then, the presence or absence of an open reading frame (ORF) for 50 or more amino acids at the forward direction of Gene 10B to be fused with a display protein in T7 SELECT10-3b was examined. As shown in table 7, in-frame ORF fusion was observed in 2 clones (clone nos. 5 and 17). In addition, since a single strand of a PCR product was subjected to sequencing, sequence accuracy was imperfect. Thus, the number of frameshift ORF of a single base was determined. As a result, it was found that there was a possibility of the occurrence of ORF fusion in 7 clones (clone nos. 2, 5, 7, 9, 10, 12, and 17).

Then, clones each containing insert DNA were subjected to homology search using translated BLASTx. Translated BLASTx is a program for homology search at the amino acid sequence level based on the matching of amino acid sequences generated through translation of query nucleotide sequences using 6 different frames with amino acid sequences obtained through translation of nucleotide sequences registered with the database of interest using 6 different frames in a similar manner. Accordingly, as shown in table 7, in the cases of about half of the clones, it was found that the amino acid sequence encoded by insert DNA was homologous to the amino acid sequence of a functional protein. Meanwhile, also in the case in which ORF was found in insert DNA, there were many insert DNAs encoding proteins having functions that were unknown due to lack of homology.

### [Example 3] Preparation of cyclodextrin-Sepharose 6B beads

As carriers for affinity beads, epoxy-activated Sepharose 6B beads (Lot. 288904, Amersham Biosciences) were used. In addition, γ-cyclodextrin (hereafter to be referred to as "γ-CD") was purchased from Wako Pure Chemical Industries, Ltd. Dry beads (3 g) were measured and made swollen with dH₂O, followed by washing. After dH₂0 was substituted with a coupling buffer (0.01 M NaOH, pH 12.4), γ-CD, the beads, and the coupling buffer were transferred to a 25-cm² flask for cell. Reaction was carried out while the flask was agitated at 45°C for 20 hours (γ-CD/ beads / buffer = 1.3 g/5 ml/10 ml → 200 µmoles of γ-CD/ml gel (in an amount 5-10 times that of active groups)). Meanwhile, β-cyclodextrin (hereafter referred to as "β-CD") and Sepharose 6B beads were subjected to coupling in a similar manner (β-CD/ beads / buffer = 0.178 g/5 ml/10 ml → 30 µmoles of β-CD/ml gel (in an amount 0.75-1.5 times that of all active groups)). In this case, β-CD in a sufficient amount could not be added due to the low solubility. Thus, unlike the case of γ-CD, a buffer containing β-CD was added by replacing it with fresh buffer after a reaction at 45°C for 20 hours. The resultant was further subjected to a reaction at 45°C for 8 hours.

After the termination of coupling, beads were loaded into a column and washed well with dH₂O such that excessive γ-CD or β-CD and the coupling buffer were completely removed. Thereafter, the solution was substituted with a blocking buffer (1 M ethanol amine, pH 8.0) and the beads were transferred back to the flask. The beads were stirred in the presence of a blocking buffer so as to be subjected to a blocking reaction at 45°C for 20 hours. After the termination of the blocking reaction, the beads were loaded into a column and washed well with dH₂O. Further, the beads were washed four times with the alternating use of washing buffer 1 (0.1 M sodium acetate (pH 4.0)/0.5 M NaCl) and washing buffer 2 (0.1 M Tris-HCl (pH 8.0)/0.5 M NaCl). At the end, the beads were washed well with dH₂O such that affinity beads (γ-CD-Sepharose 6B beads or β-CD-Sepharose 6B beads) were obtained.

γ-CD and β-CD specifically bind to cyclodextrin glucanotransferase. Thus, the binding ability of affinity beads was evaluated using roughly purified Contizyme (cyclodextrin glucanotransferase (CGTase) obtained from Amano Enzyme Inc.) (Lot. CGTRZ095184 2L). As a result, the affinity beads (per 100 µl as bed volume) were confirmed to be able to specifically bind to 200 mg of CGTase.

### [Example 4] Affinity screening

### 4-1. Materials used for affinity screening

For affinity screening, the following materials were used. Libraries #16P1 and #17P1 were used with titers of 7.6 x 10¹⁰ pfu/ml and 5.3 x 10¹⁰ pfu/ml, respectively. In addition, γ-CD-Sepharose 6B beads prepared in Example 3 were used as affinity beads used for screening. Sepharose 6B beads were used as a control.

Further, for the purpose of inhibiting nonspecific adsorption of proteins to vessel walls, a 1.5-ml siliconized tube 2150Z (Assist) was used as a vessel. As a washing buffer for screening, washing buffer 1 (25 mM Tris-HCl, pH 7.0/0.1 M NaCl/0.1 % Tween 20), washing buffer 2 (50 mM Tris-HCl, pH 7.0/ 0.2 M NaCl/ 0.2% Tween 20), and washing buffer 3 (50 mM Tris-HCl, pH 9.5/ 0.2 M NaCl) were used. For elution from beads, an elution buffer (25 mM Tris-HCl pH 7.0/0.1 M NaCl) containing 10 mM γ-CD was used.

As host cells used for T7 phage amplification, BLT5615 cells (Novagen) were used.

The PCR reagent used was made up of an Ex Taq enzyme, an Ex Taq buffer, a dNTP mix (TaKaRa), and T7 up and T7 down primers produced by SIGMA genosys on commission (originally manufactured by Novagen) were used.

A primer for sequencing analysis (TCGTATTCCAGTCAGGTGTG (SEQ ID NO: 5)) was designed and sequencing analysis of library DNA was conducted by BEX. Co., Ltd. The other reagents used were special grade reagents produced by Wako Pure Chemical Industries, Ltd., SIGMA, and the like. In addition, a variety of buffers were prepared in accordance with standard techniques, followed by sterilization using an autoclave according to need.

### 4-2. Affinity screening

γ-CD-Sepharose 6B beads (50% slurry) (40 µl) were placed in a 1.5-ml siliconized tube. Washing buffer 1 (500 µl) was added thereto, resulting in equilibration of the beads. Then, the tube was subjected to centrifugation at 12,000 rpm such that beads were precipitated. Thereafter, the supernatant thereof was removed.

Then, 50 µl of each of libraries #16P1 and #17P1 was added to a separate tube, followed by agitation using a rotary mixer at 40 rpm at room temperature for 1 hour. Thus, the libraries were allowed to adsorb to beads. One hour thereafter, the tubes were subjected to centrifugation at 12,000 rpm for 2 minutes, resulting in precipitation of beads. Then, the supernatant thereof was removed. Further, washing buffer 1 (500 µl) was added to beads, followed by agitation and washing using a Tube Mixer for 5 minutes. Five minutes thereafter, the tubes were subjected to centrifugation at 12,000 rpm for 2 minutes, resulting in precipitation of the beads. The supernatant of each thereof was removed. This washing operation was repeated 5 times.

The beads were transferred to another siliconized tube to remove the influence of phages adhering to the wall of each tube in a nonspecific manner. Then, the tubes were subjected to centrifugation at 12,000 rpm, resulting in precipitation of the beads. Thereafter, the supernatant each thereof was removed. Subsequently, phages binding to beads were eluted with the addition of 200 µl of the aforementioned elution buffer to each tube, followed by stirring using a Tube Mixer for 5 minutes. Five minutes thereafter, the tubes were subjected to centrifugation at 12,000 rpm, resulting in precipitation of beads. Then, the supernatant thereof was recovered as an eluent.

Next, the eluent was subjected to the following amplification operation. The eluent (100 µl) was added to 500 µl of BLT5615 cells induced by IPTG. Phages in the eluent were allowed to adsorb to BLT5615 cells via culture at 37°C for precisely 5 minutes. Thereafter, the cells carrying phages were subjected to centrifugation at 4,000 rpm for 2 minutes so as to be allowed to precipitate, resulting in removal of γ-CD. Next, the unnecessary supernatant thereof was removed. Then, 500 µl of an LB/Amp/IPTG medium was added to the precipitated cells. The resultant was subjected to rotation culture at 37°C until bacteriolysis occurred. Upon the occurrence of bacteriolysis, impurities were allowed to precipitate via centrifugation at 12,000 rpm for 10 minutes, such that the supernatant that was a phage solution was recovered. The phage solution (100 µl) was used for another amplification operation. Likewise, the amplification operation was repeated until Round 7. In addition, during the amplification operation for the final round (Round 7), bacteriolysis was carried out without separating cells from the supernatant thereof.

Then, the phage solution for each round was diluted for use by the following general method such that 200 clusters of T7 phage plaque appeared in a 9-cm petri dish. First, 250 µl of BLT5615 cells, 100 µl of a diluted T7 phage solution, 24 µl of a 0.5 M IPTG solution, and 3 ml of top agar heated to 50°C were mixed in a tube. Thereafter, the mixed solution was immediately laminated on a 9-cm plate containing an LB/Amp medium while the solution was kept warm. After laminated agar became solidified, the solid phase was incubated at 37°C for 2 hours, resulting in the generation of phage plaque. In addition, the titer of the phage was calculated based on the number of generated phage plaque clusters and the dilution rate.

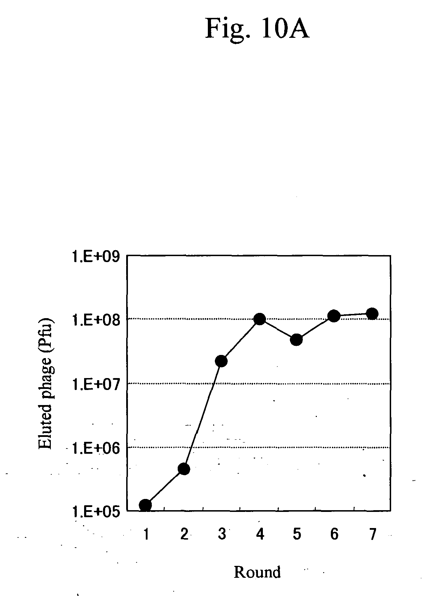

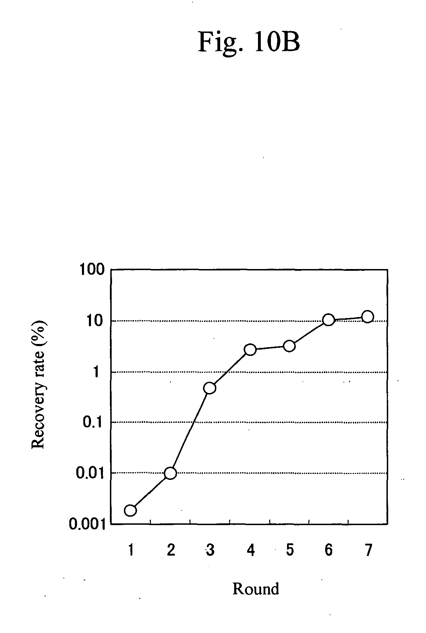

The titer of the phage solution was approximately 1 to 2 × 10¹⁰ Pfu/ml. Table 8 shows the titer of the phage solution and the recovery rate relative to the number of input phages at each round. Figs. 10A and 10B show the transition of the titer and that of the recovery rate, respectively.

**Table 8**

| Round | Eluted phage (Pfu) | Recovery rate (%) |
|---|---|---|
| 1 | 1.24E+05 | 0.0019 |
| 2 | 4.64E+05 | 0.01 |
| 3 | 2.26E+07 | 0.48 |
| 4 | 1.00E+08 | 2.8 |
| 5 | 4.80E+07 | 3.2 |
| 6 | 1.14E+08 | 10.5 |
| 7 | 1.26E+08 | 12.2 |

As is apparent from table 8 and figs. 10A and 10B, the titer of the phage solution and the recovery rate increased as the round proceeded until Round 4. After Round 4, the increase in the transition leveled off.

Then, plaque PCR was carried out to examine the level of the presence of the obtained phages containing insert DNA and the change of the insert DNA in each round for panning.

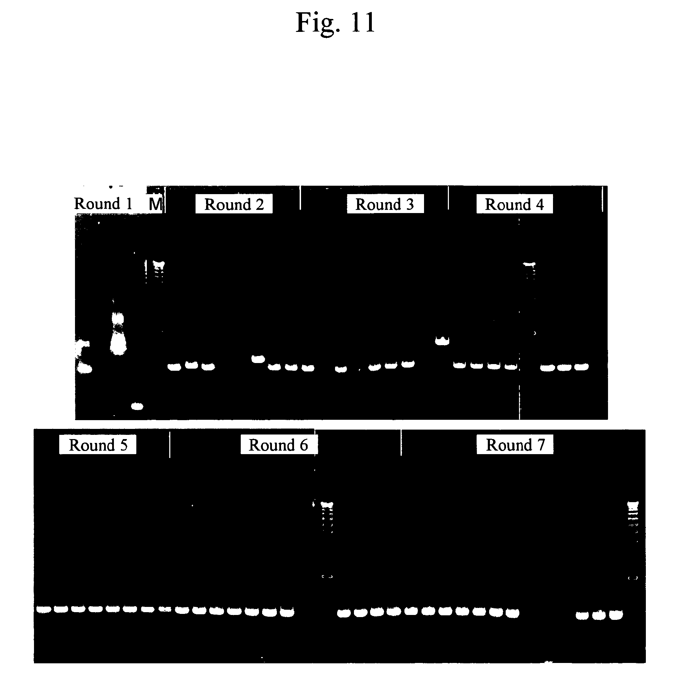

A single cluster of phage plaque on a plate was scratched with a toothpick. The obtained plaque was suspended in 15 to 20 µl of a PCR Reaction Mixture (containing a 0.1 pmol of primers and 0.03 U of Ex Taq enzyme). In addition, the primers used were T7 up and T7 down primers (Novagen). PCR reaction was carried out using a thermal cycler under the following conditions: 1) 1 cycle at 96°C for 3 minutes; 2) 33 cycles at 94°C for 30 seconds, at 50°C for 30 seconds, and at 72°C for 2 minutes, and 3) 1 cycle at 72°C for 10 minutes. After the termination of the reaction, 1 µl of BPB dye was added to 5 µl of a PCR sample, followed by 0.8% agarose gel electrophoresis at 100 V for 35 minutes. Then, comparison in terms of insert DNA in phages was carried out. The results are shown in fig. 11. In fig. 11, each lane indicates a plaque PCR product of the phage solution obtained in each round.

As is apparent from fig. 11, during Rounds 1 to 3, a single insert DNA having a different size was found in several insert DNAs. However, after Round 4, most bands of the same insert DNAs had sizes of 0.7 Kbp. The results indicated that concentration of a specific clone occurred as a result of affinity selection.

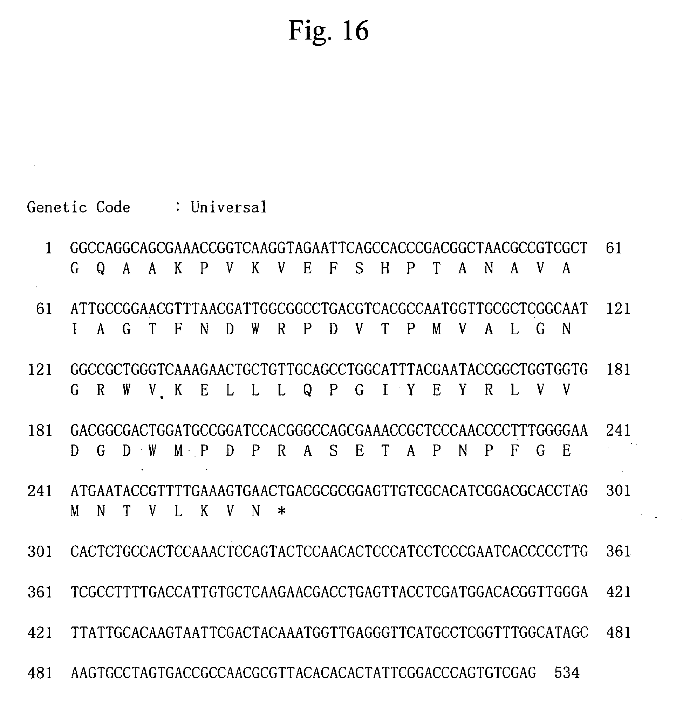

As a result of sequencing of the nucleotide sequence of 0.7 kbp insert DNA (SEQ ID NO: 6), an ORF consisting of 88 amino acids (SEQ ID NO: 7) was confirmed (fig. 16). The clone and the ORF were designated as P31.

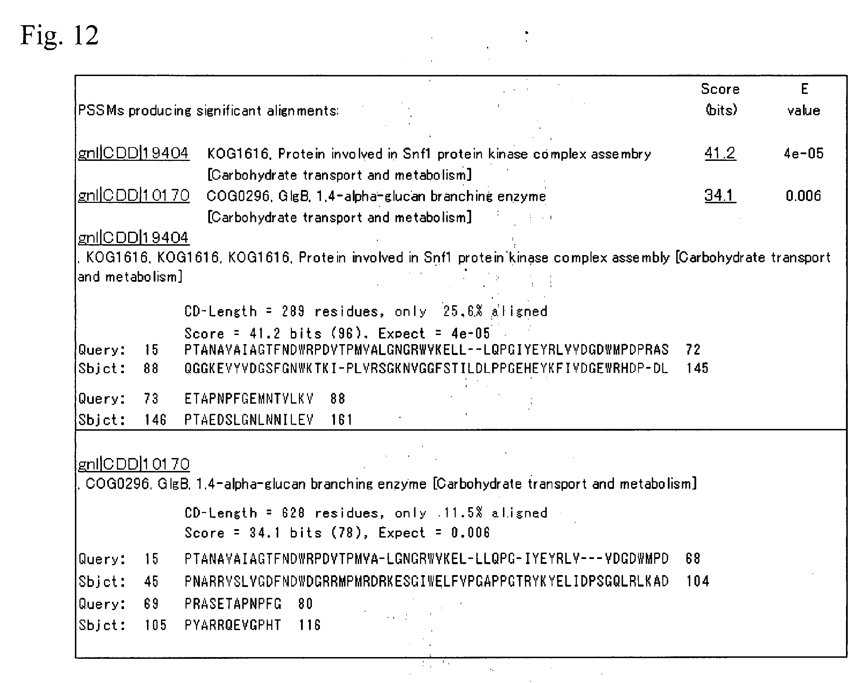

Regarding P31, database search was carried out using a BLAST homology search program (http://www.ncbi.nlm.nih.gov/BLAST) of the NCBI (National Center for Biology Information) in the U.S. The results are shown in fig. 12. As is apparent from fig. 12, P31 was found to hit two candidates in the conserved sequence database (Conserved Domain DB). One candidate is a protein of Snf1 protein kinase complex assembly, which is involved in transportation and metabolism of carbohydrates, and another one is 1,4-α-glucan branching enzyme. Both of the hit proteins had a function of recognizing a carbohydrate.

The protein of Snf1 protein kinase complex assembly and P31 have the same 24 amino acids out of 73 amino acids in the alignment portion, so as to have 33% homology to each other. In addition, when analogous amino acids are included, they have the same 37 amino acids so as to have 50% homology to each other. Accordingly, based on the homology to conserved sequences, it has been strongly suggested that P31 is a part of a protein having a function of recognizing saccharide.

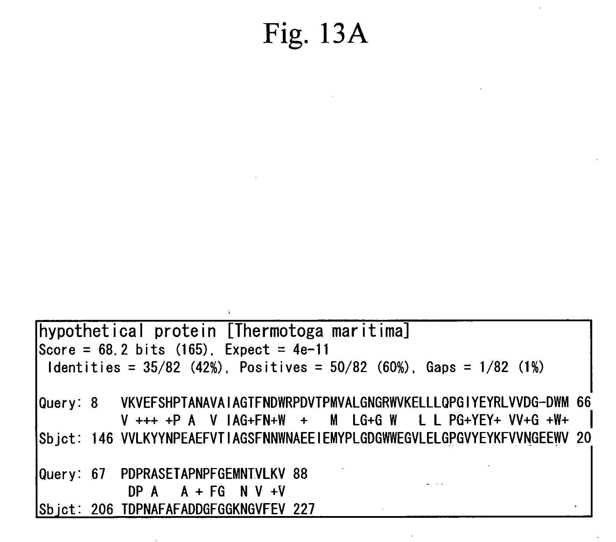

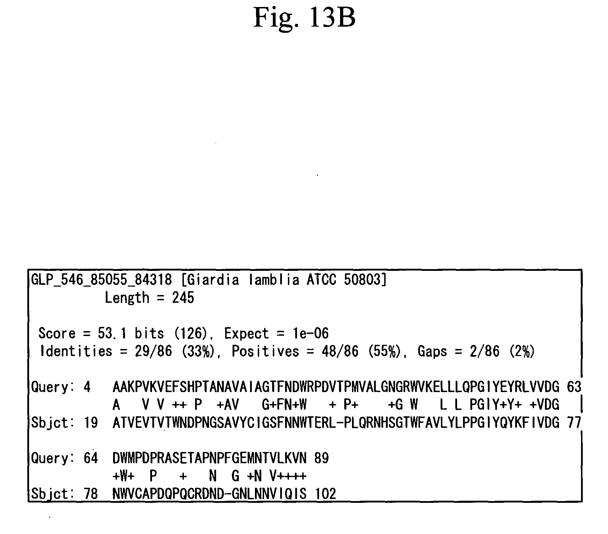

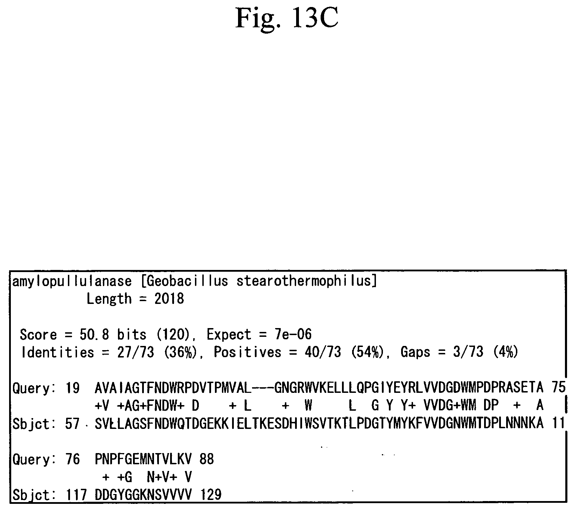

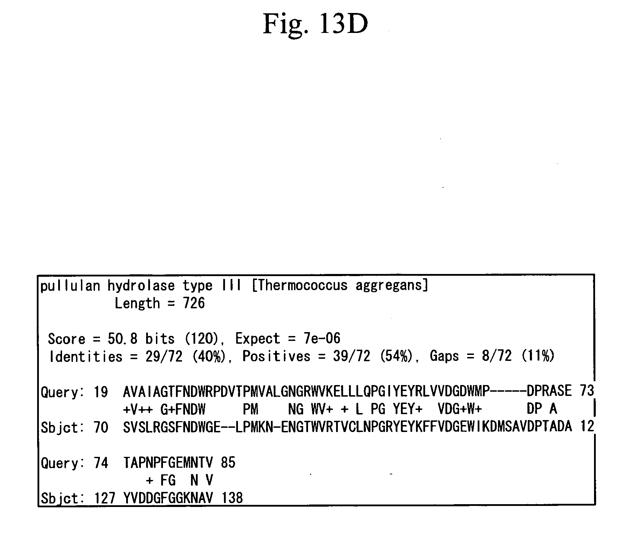

Further, Blastp searches were carried out in all sequence databases. Figs. 13A to 13D show the alignments of the above four candidates with p31. Figs. 13A to 13D show the alignment results, in which the E (Expect) value is a number between 0 and 1 and a parameter indicating a percentage of coincidence. When the E value is the closest to 0, the highest level of reliability is obtained. In general, when a hit is obtained at an E value of approximately 10⁻¹⁰, a protein of interest is believed to have a significant high probability of having a function similar to the protein of the hit sequence. The hypothetical protein (Thermotoga maritima) shown in fig. 13A, which had the highest homology to P31, had an E value of 4 x 10⁻¹¹. Thus, it has been suggested that there is a high probability that a protein containing P31 has a function similar to that of such hypothetical protein. In addition,- a hypothetical protein shown in fig. 13A is assumed to be an O-type sugar chain-degrading enzyme (see http://www.ebi.ac.uk/ego/DisplayGoTerm?id=GO:0004553). Further, GLP_546_85055_84318 (Giardia lamblia) ATCC 50803 shown in fig. 13B had the second highest homology to P31 and comprised a motif of AMP kinase associated with isoamylase (see http://kr.expasy.org/cgi-bin/niceprot.pl?Q7R2K2). Furthermore, amylopullulanase (Geobacillus stearothermophilus) and pullulan hydrolase type III (Thermococcus aggregans) shown in figs 13C and 13D had the third and fourth highest homologies to P31, respectively, and they were both examples of amylopullulanase, which is a 1,4-α-glucan branching enzyme.

Accordingly, it has been suggested that a protein containing P31 has a function of recognizing saccharide.

### [Example 5] Ligand binding profile of P31

The obtained P31-presenting phage clone was purified. Then, a γ-CD binding profile was examined.

A lysate of the purified P31-presenting phage clone (100 µl), 100 µl of a W1T buffer (25 mM Tris-HCl pH 7.0, 0.1 M NaCl, and 0.1% Tween 20), and 20 µl of γ-CD-Sepharose 6B beads equilibrated with a W1T buffer were mixed, followed by shaking at room temperature for 1 hour. Subsequently, the mixed solution was subjected to centrifugation at 12000 rpm for 2 minutes. Then, the supernatant was removed. Further, 500 µl of a W1T buffer was added to γ-CD-Sepharose 6B beads, followed by shaking using a mixer for 5 minutes. The mixed solution was subjected to centrifugation at 12000 rpm for 2 minutes, followed by washing. This washing operation was repeated 5 times. Herein, the last washing operation was carried out using a Tween-free W1 buffer (25 mM Tris-HCl pH 7.0, 0.1 M NaCl).

After washing, 80 mM of γ-CD dissolved in 100 µl of a W1 buffer was added to γ-CD-Sepharose 6B beads, followed by mixing for 5 minutes. Thereafter, the mixed solution was subjected to centrifugation at 12000 rpm for 5 minutes. Then, the supernatant was recovered.

Then, the concentration of phages existing in the supernatant was calculated via plaque assay. Further, a binding test in the presence of 1% starch was carried out using phage clones presenting P31 and γ-CD-Sepharose 6B beads. The starch used was prepared by dissolving a 10% suspension of soluble starch (Wako Pure Chemical Industries, Ltd.) during heating at 60°C. Then, the dissolved solution was added in a volume 1/10 that of the same. In addition, likewise, a β-CD binding profile of a P31-presenting phage clone was examined.

As a control with respect to a P31-presenting phage clone, wild type phage T7SC1 was used. Further, Sepharose 6B beads (S6B) were used as a control with respect to γ-CD-Sepharose 6B beads and β-CD-Sepharose 6B beads.

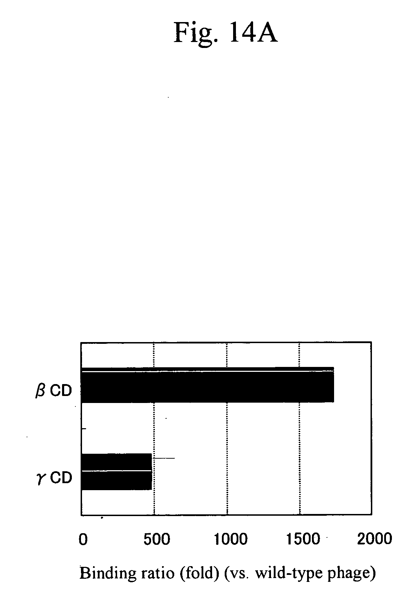

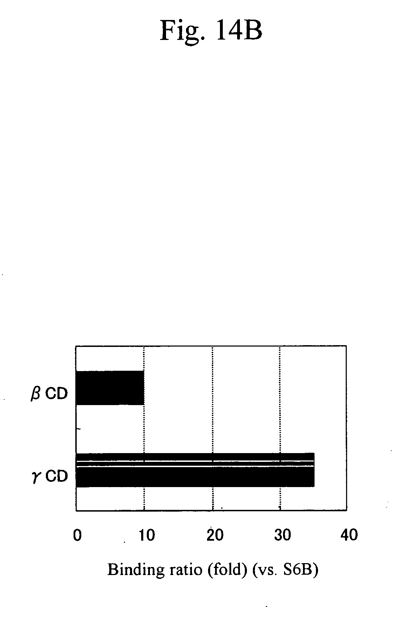

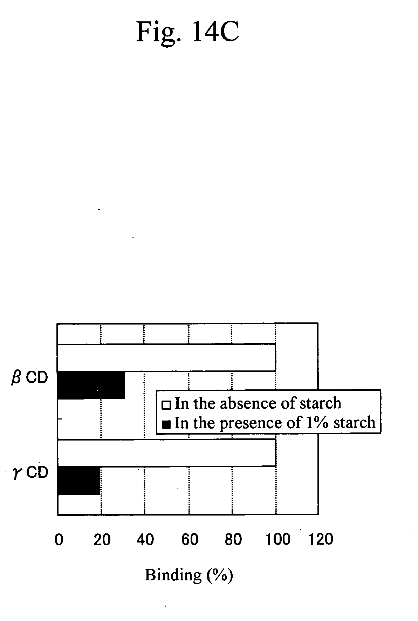

The results are shown in tables 9 and 10 and figs. 14A to 14C. Table 9 shows the bindings of P31-presenting phage clones to β-CD-Sepharose 6B beads. Meanwhile, table 10 shows the bindings of P31-presenting phage clones to γ-CD-Sepharose 6B beads. Fig. 14A shows the bindings of P31-presenting phage clones to β- and γ-CD-Sepharose 6B beads compared with wild type phages T7SC1 as controls. Fig. 14B shows the bindings of P31-presenting phage clones to β- and γ-CD-Sepharose 6B beads compared with Sepharose 6B beads (S6B) as controls. Further, fig. 14C shows inhibition due to 1% starch of the bindings of P31-presenting phage clones to β- and γ-CD-Sepharose 6B beads.

**Table 9**

| Phage | Bead | Treatment | (PFU) Input | (PFU) Elution | Recovery rate |
|---|---|---|---|---|---|
| P31 | S6B | | 4.00E+08 | 1.40E+06 | 0.0350% |
| P31 | βCD | | 4.00E+08 | 1.40E+07 | 0.3475% |
| P31 | βCD | 1% starch | 4.00E+08 | 4.40E+06 | 0.1100% |
| T7SC1 | βCD | | 4.60E+09 | 3.00E+05 | 0.0002% |

**Table 10**

| Phage | Bead | Treatment | (PFU) Input | (PFU) Elution | Recovery rate |
|---|---|---|---|---|---|
| P31 | S6B | | 8.50E+08 | 2.60E+06 | 0.02740% |
| P31 | γ-CD | | 8.50E+08 | 9.20E+07 | 0.96840% |
| P31 | γ-CD | 1% starch | 8.50E+08 | 1.80E+07 | 0.18950% |
| T7SC1 | γ-CD | | 4.60E+09 | 4.60E+05 | 0.00200% |

As shown in fig. 14A, P31-presenting phage clones bound to β-CD-Sepharose 6B beads and to γ-CD-Sepharose 6B beads at levels approximately 1700 and 450 times, respectively, greater than in the case of wild type phages as controls. In addition, as shown in fig. 14B, P31-presenting phage clones bound to β-CD-Sepharose 6B beads and to γ-CD-Sepharose 6B beads at levels 10 and 35 times, respectively, greater than in the case of Sepharose 6B beads (S6B) as controls. Further, as shown in fig. 14C, the specific binding of a P31-presenting phage clone to β-CD-Sepharose 6B beads and to γ-CD-Sepharose 6B beads was inhibited by 70% to 80% due to the presence of 1% starch. A difference between the wild type phage and the P31 clone involved an inserted portion. A difference between the S6B beads as a control and the CD beads involved the presence or absence of a CD portion. Both CD and starch have 1,4-α-glucan. Accordingly, it has been shown that the binding of a protein expressed by an insert of P31 is based on not only sequence homology but also on recognition of 1,4-α-glucan.

### [Example 6] Obtaining of fragments of a novel aldose epimerase-like gene

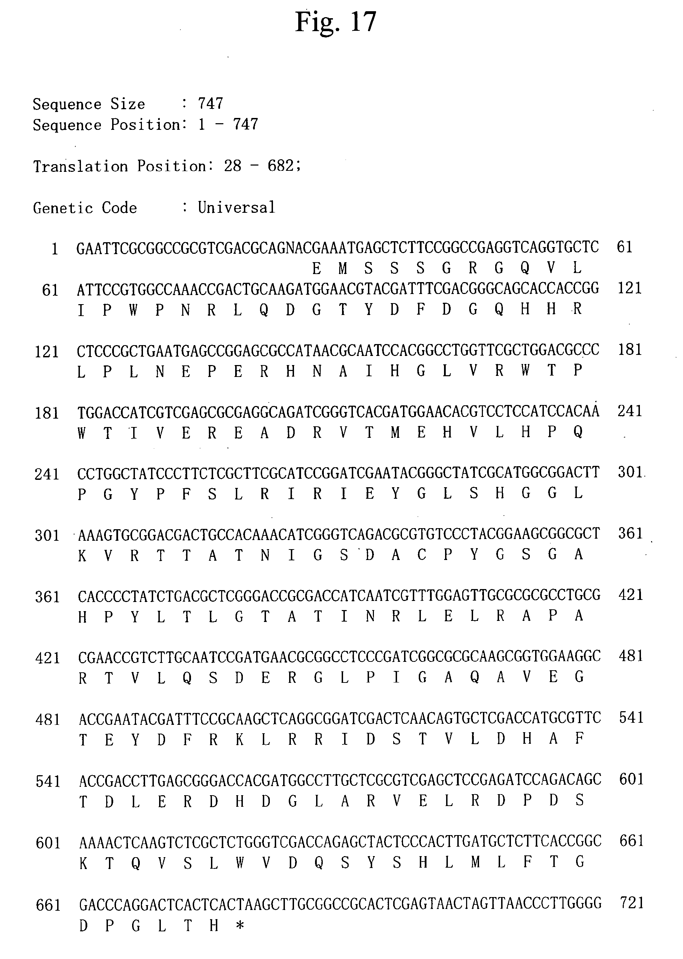

The phage solution after Round 3 of panning obtained in Example 4 above was subjected to plating. The nucleotide sequence of insert DNA of a single plaque that had been randomly selected was examined so that sequences highly homologous to aldose-1-epimerase were found (fig. 17: SEQ ID NOS: 8 and 9). The clone was designated as AE1.

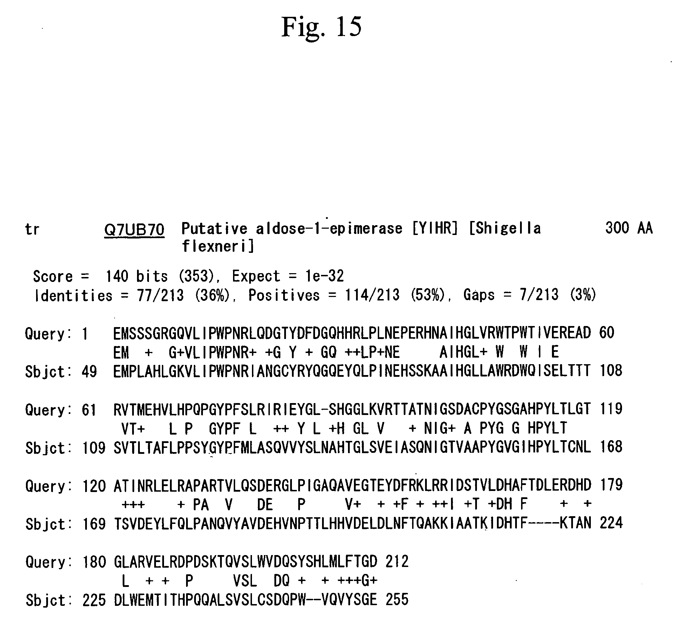

Regarding the sequence, as described above, database search was carried out using a BLASTp homology search program (http://www.ncbi.nlm.nih.gov/BLAST) of the NCBI (National Center for Biology Information). Fig. 15 shows the results. As shown in fig. 15, the amino acid sequence (SEQ ID NO: 9) of a protein encoded by a gene containing insert DNA (SEQ ID NO: 8) in AE1 had homology to an amino acid sequence called QUB70 (Putative aldose-1-epimerase (EC 5.1.3.3) (YIHR) (Shigella flexneri)) at an extremely low E value (1 x 10⁻³²) . Thus, it was suggested that the gene containing insert DNA (SEQ ID NO: 8) in AE1 was a novel aldose-1-epimerase gene. In addition, aldose-1-epimerase is an enzyme converting α-D-glucose into β-D-glucose. Therefore, it was considered that AE1 bound to beads while recognizing glucose constituting CD or amylose.

Then, the binding of AE1 to amylose resin (New England Biolabs) was examined in a manner similar to that used for examination of the binding of a P31-presenting phage clone to CD-Sepharose 6B beads in Example 5. Elution was carried out using 20 mM maltose. In addition, a wild type phage T7SC1 was used as a control phage. Table 11 shows the results.

**Table 11**

| Phage | Resin | (PFU) Input | (PFU) Elution | Recovery rate |
|---|---|---|---|---|
| T7SC1 | Amylose | 2.40E+08 | 8.00E+04 | 0.03% |
| AE1 | Amylose | 4.50E+07 | 1.01E+06 | 2.24% |

As shown in table 11, AE1 bound to amylose resin at a level 67 times greater than the case of wild type phage T7SC1. Thus, it was suggested, consistently with the above results of sequence homology, that a gene product containing insert DNA in AE1 recognizes glucose constituting cyclodextrin or amylose so as to bind to beads.

Round 3 in accordance with the above method ended after 2 days. The obtaining and analysis of a single clone ended after around 3 days. Thus, as described above, it has been shown that fragments of a group of functional protein genes that recognize ligands can be cloned from unused genetic resources in soil in a short period of time with good efficiency via a cycle of affinity enrichment using ligands based on a metagenome display library without the need for culture steps.

### [Example 7] Affinity screening using amylose magnetic beads

Escherichia coli BLT5615 (1.5 ml) that had been induced using 1mM IPTG was infected with 15 µl of library #17P1, followed by shaking culture for 1 hour. Thus, the lysate thereof was obtained. The obtained lysate was subjected to centrifugation at 10,000 x g for 10 minutes. After centrifugation, the obtained supernatant was designated as library # 17P2.

Library #17P2 (400 µl) was subjected to centrifugation 3 times using Amicon microcon YM-100 (cutoff: Mw 100,000) such that low-molecular-weight substances that could compete with ligands used for panning were removed, and substituted with buffer W2T (25 mM Tris-HCl, pH 7.0, 0.2 M NaCl, 0.1% Tween20) so that 200 µl of the resultant was obtained. Amylose magnetic beads (1 mg) (New England Biolabs) blocked by W2TgR (W2T+0.2% gelatin, 200 µg/ml yeast RNA) were added thereto. The resulting suspension was allowed to stand for 1 hour such that the beads and phages having affinity to amylose were subjected to a binding reaction. Thereafter, beads and the reaction solution were separated from each other using a strong magnet (Dynal MPC-S). Then, 500 µl of W2TgR was added thereto. The resulting suspension was allowed to stand for 5 minutes, resulting in separation of the beads. This operation was repeated 4 times. Then, 500 µl of W2TgR was added thereto. The resulting suspension was allowed to stand for 5 minutes, resulting in separation of the beads. Then, the resultant was recovered.

Escherichia coli BLT5615 (1 ml) that had been induced using 1mM IPTG was infected with 250 µl of the recovered resultant. The lysate was treated as described above. Then, the 2^{nd} cycle was carried out. After washing 4 times, 500 µl of W2TgR was added. The resulting suspension was allowed to stand for 5 minuets. Then, the obtained fraction (approximately 5 x 10⁵ pfu) was recovered.

The recovered fraction was adequately diluted. Then, plaque assay was carried out in a manner similar to that used for Example 4. Further, plaque PCR was carried out. The nucleotide sequence of an insert obtained by plaque PCR was examined in relation to 8 clones. Accordingly, as sugar-related enzymes having high homology, the two following types of gene fragments were obtained both of which were considered to be of the E.C.3.2.1 family member β-xylosidase, glucosidase (TrEMBL accession #Q9A3Z6, caulobactor crescentus xylosidase, homology 48%, E = 8E-51), and the E.C.3.2.1.3, glucoamylase (TrEMBL accession #Q98EK2, Rhizobium loti glucoamylase, homology 36%, E = 3E-11), respectively.

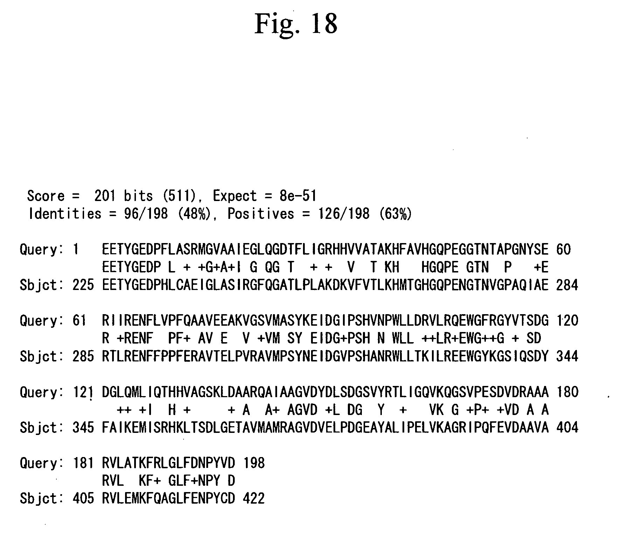

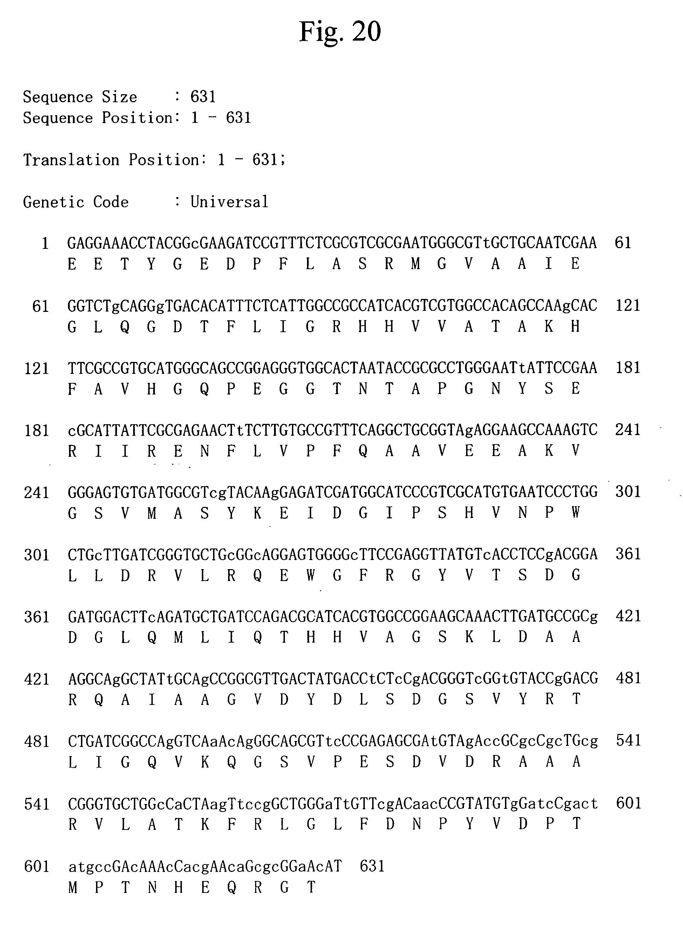

Fig. 18 shows an alignment of the amino acid sequence of the E.C.3.2.1 family member β-xylosidase, glucosidase (TrEMBL accession #Q9A3Z6, caulobactor crescentus xylosidase, homology 48%, E = 8E-51) and the amino acid sequence of a protein (SEQ ID NO: 11: amino acid sequence of a clone homologous to β-glucosidase) encoded by one of the obtained gene fragments (SEQ ID NO: 10: nucleotide sequence of a clone homologous to β-glucosidase). In addition, in fig. 18, the Query sequence is the amino acid sequence of a protein encoded by the obtained gene fragment, and the Sbjct sequence is the amino acid sequence of the E.C.3.2.1 family member β-xylosidase, glucosidase. In addition, fig. 20 shows the nucleotide sequence of the gene fragment (SEQ ID NO: 10) and the amino acid sequence of a protein encoded by the nucleotide sequence (SEQ ID NO: 11).

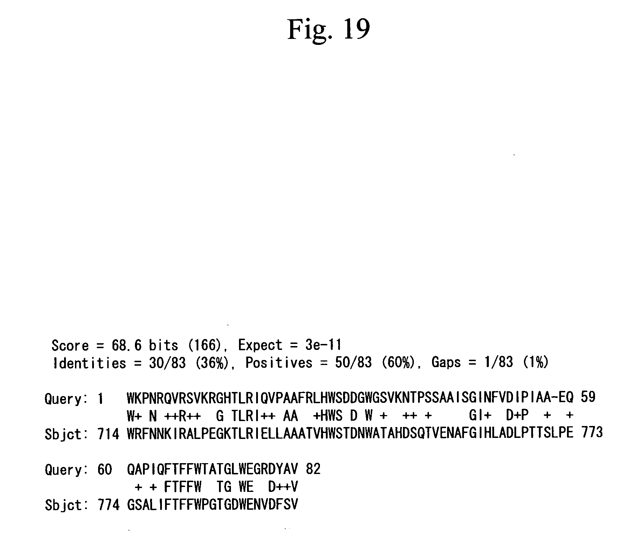

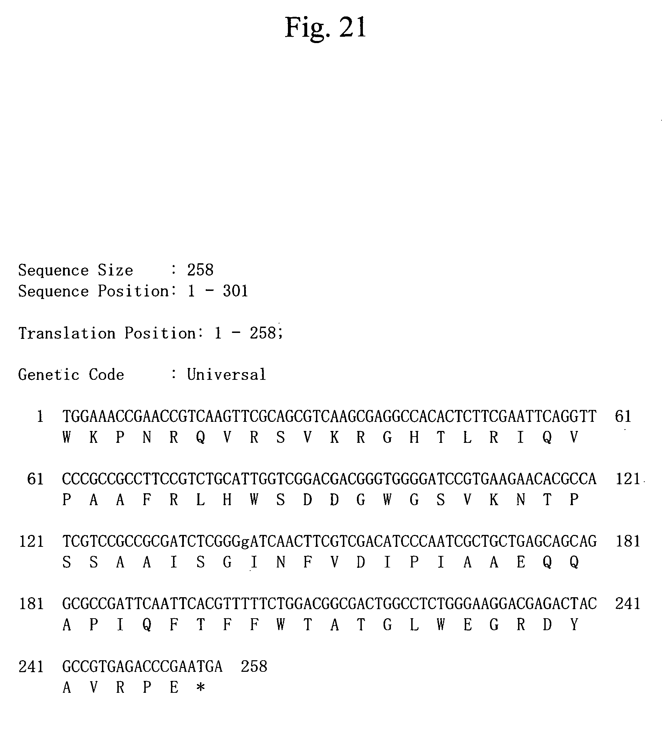

Further, fig. 19 shows an alignment of the amino acid sequence of the E.C.3.2.1.3, glucoamylase (TrEMBL accession #Q98EK2, Rhizobium loti glucoamylase, homology 36%, E = 3E-11) and the amino acid sequence (SEQ ID NO: 13: amino acid sequence of a clone homologous to glucoamylase) of a protein encoded by the other gene fragment (SEQ ID NO: 12: nucleotide sequence of a clone homologous to glucoamylase). In addition, in fig. 19, the Query sequence is the amino acid sequence of a protein encoded by the obtained gene fragment, and the Sbjct sequence is the amino acid sequence of the E.C.3.2.1.3, glucoamylase. Further, fig. 21 shows the nucleotide sequence of the gene fragment (SEQ ID NO: 12) and the amino acid sequence of a protein encoded by the nucleotide sequence (SEQ ID NO: 13).

### [Example 8] Affinity screening using streptavidin magnetic beads

Library #17P2 (400 µl) obtained in Example 7 was subjected to centrifugation 3 times using Amicon microcon YM-100 (cutoff: Mw 100,000) such that low-molecular-weight substances that could compete with ligands used for panning were removed, and substituted with buffer W2T (25 mM Tris-HCl, pH 7.0, 0.2 M NaCl, 0.1% Tween20) so that 200 µl of the resultant was obtained. 2mM α1-3,α1-6-D-mannotriose-biotin (10 µl) (14-atom spacer, DEXTRA Laboratories, Ltd.) was added thereto, followed by mixing. The resultant was allowed to stand at room temperature for 1.5 hours. Then, streptavidin magnetic beads (1 mg) (New England Biolabs) blocked by W5TgR (25 mM Tris-HCl, pH7.0, 0.5 M NaCl, 0.1% Tween20, 0.2% gelatin, 200 µg/ml yeast RNA) were added thereto. The resulting suspension was allowed to stand for 1 hour such that the beads and phages having affinity were subjected to a binding reaction. Thereafter, the beads and the reaction solution were separated from each other using a magnet (Dynal MPC-S). Then, 500 µl of W5TgR was added thereto. The resulting suspension was allowed to stand for 5 minutes, resulting in separation of the beads. This operation was repeated 5 times. Then, 200 µl of W5TgR containing 10 mM mannose was added thereto. The resulting suspension was allowed to stand for 5 minutes, resulting in separation of the beads. Then, the supernatant thereof was recovered.

The recovered supernatant was adequately diluted. Then, plaque assay was carried out in a manner similar to that used for Example 4. Further, plaque PCR was carried out. The nucleotide sequence of an insert obtained by plaque PCR was examined in relation to 7 clones. Accordingly, as a sugar-related enzyme having high homology, one type of a gene fragment was obtained, which was considered to be E.C.4.2.1.16, dTDP glucose dehydratase (TrEMBL accession #Q5V3C6, Haloarcula marismortui, dTDP-glucose 4,6-dehydratase, homology 66%, E = 4E-59).

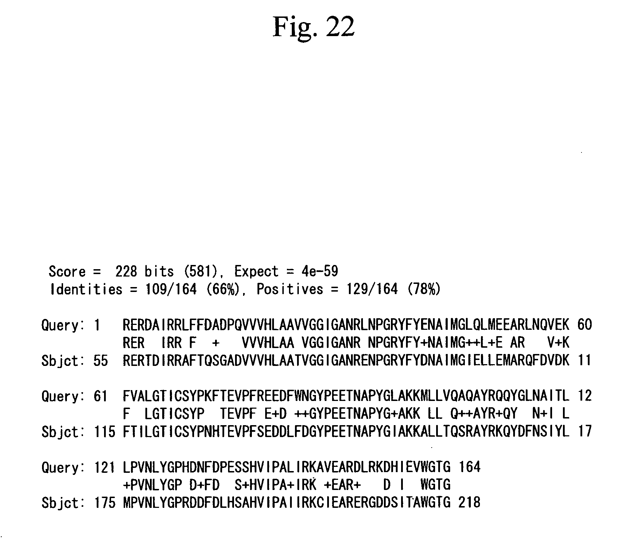

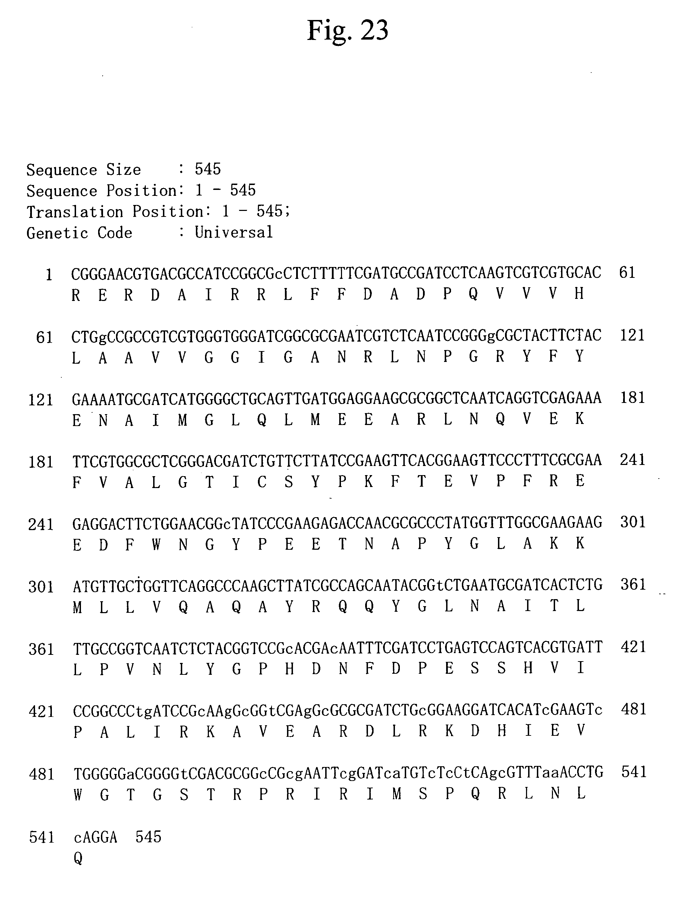

Fig. 22 shows an alignment of the amino acid sequence of the E.C.4.2.1.16, dTDP glucose dehydratase (TrEMBL accession #Q5V3C6, Haloarcula marismortui, dTDP-glucose 4,6-dehydratase, homology 66%, E = 4E-59) and the amino acid sequence (SEQ ID NO: 15: amino acid sequence of a clone homologous to glucose dehydratase) of a protein encoded by the obtained gene fragment (SEQ ID NO: 14: the nucleotide sequence of a clone homologous to glucose dehydratase). In addition, in fig. 22, the Query sequence is the amino acid sequence of a protein encoded by the obtained gene fragment, and the Sbjct sequence is the amino acid sequence of the E.C.4.2.1.16, dTDP glucose dehydratase. Further, fig. 23 shows the nucleotide sequence of the gene fragment (SEQ ID NO: 14) and the amino acid sequence of a protein encoded by the nucleotide sequence (SEQ ID NO: 15).

All publications, patents, and patent applications cited herein are incorporated herein by reference in their entirety.

### Industrial Applicability

In accordance with the method of purifying environmental DNA derived from an environmental sample of the present invention, high-purity DNA derived from an environmental sample can be obtained. Since the obtained DNA derived from an environmental sample is high-purity, it can be used for many forms of genetic engineering treatment. Meanwhile, in accordance with the method of screening for protein-encoding genes of the present invention, screening can be carried out by enriching (improving the S/N ratio) a group of protein-encoding genes of interest based on a display library created from environmental DNA derived from an environmental sample using desired ligands in a short period of time in a labor-saving manner.

### Free Text of Sequence Listing

SEQ ID NOS: 1-4 represent synthetic oligonucleotides.
SEQ ID NO: 5 represents a primer.
SEQ ID NO: 6 represents the nucleotide sequence of a P31-encoding gene.
SEQ ID NO: 7 represents the amino acid sequence of P31.
SEQ ID NO: 8 represents the nucleotide sequence of a gene containing insert DNA in AE1.
SEQ ID NO: 9 represents the amino acid sequence of a protein encoded by a gene containing insert DNA in AE1.
In SEQ ID NO: 8, "n" denotes "a," "g," "c," or "t" (located at positions 25 and 84).
SEQ ID NO: 10 represents the nucleotide sequence of a clone homologous to β-glucosidase.
SEQ ID NO: 11 represents the amino acid sequence of a clone homologous to β-glucosidase.
SEQ ID NO: 12 represents the nucleotide sequence of a clone homologous to glucoamylase.
SEQ ID NO: 13 represents the amino acid sequence of a clone homologous to glucoamylase.
SEQ ID NO: 14 represents the nucleotide sequence of a clone homologous to glucose dehydratase.
SEQ ID NO: 15 represents the amino acid sequence of a clone homologous to glucose dehydratase.

## Claims

1. A method of screening for protein-encoding genes, comprising creating a display library of environmental DNA derived from an environmental sample and carrying out screening based on the interaction of the library with ligands.

2. The method of screening for protein-encoding genes according to claim 1, **characterized in that** the environmental DNA derived from an environmental sample is purified by a method of purifying environmental DNA derived from an environmental sample, comprising recovering DNA from an environmental sample and allowing the DNA to be subjected to gel filtration chromatography and/or ion exchange chromatography.

3. The method of screening for protein-encoding genes according to claim 2, **characterized in that** the method of purifying environmental DNA derived from an environmental sample comprises recovering DNA by disrupting the environmental sample using glass beads.

4. The method of screening for protein-encoding genes according to claim 2, wherein the method of purifying environmental DNA derived from an environmental sample comprises recovering DNA from an environmental sample and allowing the recovered DNA to be subjected to partial degradation.

5. The method of screening for protein-encoding genes according to claim 2, **characterized in that** the exclusion limit of the gel filtration chromatography is 10⁵ Da or more.

6. The method of screening for protein-encoding genes according to claim 2, **characterized in that** the size of DNA purified by the method of purifying environmental DNA derived from an environmental sample is 0.5 to 5 Kbp.

7. The method of screening for protein-encoding genes according to claim 1, **characterized in that** the ligands are saccharide.

8. The method of screening for protein-encoding genes according to claim 7, **characterized in that** the saccharide is selected from the group consisting of cyclodextrin, amylose, and acarbose.

9. The method of screening for protein-encoding genes according to claim 1, comprising the step of immobilizing ligands to immobilization carriers before the screening.

10. A method of screening for a clone of interest based on the expressed activity of an insert gene, comprising amplifying a DNA fragment enriched by the method of screening for protein-encoding genes according to claim 1, transferring the amplified fragments to an expression vector, and introducing the vector into a host.

11. The method of screening for protein-encoding genes according to claim 1 using a combination of two or more types of ligands and/or carriers.

12. A method of purifying environmental DNA derived from an environmental sample, comprising recovering DNA from an environmental sample and allowing the DNA to be subjected to gel filtration chromatography and/or ion exchange chromatography.

13. The method of purifying environmental DNA derived from an environmental sample according to claim 12, **characterized in that** DNA is recovered by disrupting an environmental sample using glass beads.

14. The method of purifying environmental DNA derived from an environmental sample according to claim 12, comprising recovering DNA from an environmental sample and allowing the recovered DNA to be subjected to partial degradation.

15. The method of purifying environmental DNA derived from an environmental sample according to claim 12, **characterized in that** the exclusion limit of the gel filtration chromatography is 10⁵ Da or more.

16. The method of purifying environmental DNA derived from an environmental sample according to claim 12, **characterized in that** the size of the purified DNA is 0.5 to 5 Kbp.


SEQUENCE LISTING

<110> TOAGOSEI CO., LTD.

<120> METHOD OF PURIFYING ENVIRONMENTAL DNA AND METHOD OF EFFICIENTLY
SCREENING FOR PROTEIN-ENCODING GENE FROM ENVIRONMENTAL DNA

<130> PH-2506-PCT

<140> PCT/JP2005/010907
<141> 2005-06-08

<150> JP 2004-170161
<151> 2004-06-08

<160> 15

<170> PatentIn Ver. 2.1

<210> 1
<211> 19
<212> DNA
<213> Artificial Sequence

<220>
<223> Description of Artificial Sequence:synthetic
      oligonucleotide

<400> 1
agcttagtga gtgagtcct                                                19

<210> 2

<211> 15

<212> DNA

<213> Artificial Sequence

<220>

<223> Description of Artificial Sequence:synthetic

oligonucleotide

<400> 2

aggactcact cacta 15

<210> 3

<211> 15

<212> DNA

<213> Artificial Sequence

<220>

<223> Description of Artificial Sequence:synthetic

oligonucleotide

<400> 3

gtcgacgcgg ccgcg 15

<210> 4

<211> 19

<212> DNA
<213> Artificial Sequence

<220>
<223> Description of Artificial Sequence:synthetic
      oligonucleotide

<400> 4
aattcgcggc cgcgtcgac                                                19

<210> 5
<211> 20
<212> DNA
<213> Artificial Sequence

<220>
<223> Description of Artificial Sequence:primer

<400> 5
tcgtattcca gtcaggtgtg                                               20

<210> 6
<211> 534
<212> DNA
<213> Unknown Organism

<220>
<223> Description of Unknown Organism: nucleotide sequence of a gene

encoding P31

<400> 6
ggccaggcag cgaaaccggt caaggtagaa ttcagccacc cgacggctaa cgccgtcgct 60
attgccggaa cgtttaacga ttggcggcct gacgtcacgc caatggttgc gctcggcaat 120
ggccgctggg tcaaagaact gctgttgcag cctggcattt acgaataccg gctggtggtg 180
gacggcgact ggatgccgga tccacgggcc agcgaaaccg ctcccaaccc ctttggggaa 240
atgaataccg ttttgaaagt gaactgacgc gcggagttgt cgcacatcgg acgcacctag 300
cactctgcca ctccaaactc cagtactcca acactcccat cctcccgaat cacccccttg 360
tcgccttttg accattgtgc tcaagaacga cctgagttac ctcgatggac acggttggga 420
ttattgcaca agtaattcga ctacaaatgg ttgagggttc atgcctcggt ttggcatagc 480
aagtgcctag tgaccgccaa cgcgttacac acactattcg gacccagtgt cgag 534

<210> 7
<211> 88
<212> PRT
<213> Unknown Organism

<220>
<223> Description of Unknown Organism: amino acid sequence of P31

<400> 7
Gly Gln Ala Ala Lys Pro Val Lys Val Glu Phe Ser His Pro Thr Ala
1               5                   10                  15

Asn Ala Val Ala Ile Ala Gly Thr Phe Asn Asp Trp Arg Pro Asp Val
            20                  25                  30

Thr Pro Met Val Ala Leu Gly Asn Gly Arg Trp Val Lys Glu Leu Leu

        35                  40                  45

Leu Gln Pro Gly Ile Tyr Glu Tyr Arg Leu Val Val Asp Gly Asp Trp

    50                  55                  60

Met Pro Asp Pro Arg Ala Ser Glu Thr Ala Pro Asn Pro Phe Gly Glu

65                  70                  75                  80

Met Asn Thr Val Leu Lys Val Asn

                85

<210> 8

<211> 747

<212> DNA

<213> Unknown Organism

<220>

<221> modified base

<222> 25 and 84

<223> n represents a,g,c or t

<220>

<223> Description of Unknown Organism: nucleotide sequence of a gene

including insert DNA within AE1

<400> 8

gaattcgcgg ccgcgtcgac gcagnacgaa atgagctctt ccggccgagg tcaggtgctc 60

cctctaaacg ggtctggagg ggtnaacatt ccgtggccaa accgactgca agatggaacg 120

tacgatttcg acgggcagca ccaccggctc ccgctgaatg agccggagcg ccataacgca 180

atccacggcc tggttcgctg gacgccctgg accatcgtcg agcgcgaggc agatcgggtc 240

acgatggaac acgtcctcca tccacaacct ggctatccct tctcgcttcg catccggatc 300

gaatacgggc tatcgcatgg cggacttaaa gtgcggacga ctgccacaaa catcgggtca 360

gacgcgtgtc cctacggaag cggcgctcac ccctatctga cgctcgggac cgcgaccatc 420

aatcgtttgg agttgcgcgc gcctgcgcga accgtcttgc aatccgatga acgcggcctc 480

ccgatcggcg cgcaagcggt ggaaggcacc gaatacgatt tccgcaagct caggcggatc 540

gactcaacag tgctcgacca tgcgttcacc gaccttgagc gggaccacga tggccttgct 600

cgcgtcgagc tccgagatcc agacagcaaa actcaagtct cgctctgggt cgaccagagc 660

tactcccact tgatgctctt caccggcgac ccaggactca ctcactaagc ttgcggccgc 720

actcgagtaa ctagttaacc cttgggg 747

<210> 9

<211> 217

<212> PRT

<213> Unknown Organism

<220>

<223> Description of Unknown Organism: amino acid sequence of a protein

encoded by a gene including insert DNA within AE1

<400> 9

Glu Met Ser Ser Ser Gly Arg Gly Gln Val Leu Ile Pro Trp Pro Asn

1               5                   10                  15

Arg Leu Gln Asp Gly Thr Tyr Asp Phe Asp Gly Gln His His Arg Leu

            20                  25                  30

Pro Leu Asn Glu Pro Glu Arg His Asn Ala Ile His Gly Leu Val Arg

        35                  40                  45

Trp Thr Pro Trp Thr Ile Val Glu Arg Glu Ala Asp Arg Val Thr Met
    50                  55                  60

Glu His Val Leu His Pro Gln Pro Gly Tyr Pro Phe Ser Leu Arg Ile
65                  70                  75                  80

Arg Ile Glu Tyr Gly Leu Ser His Gly Gly Leu Lys Val Arg Thr Thr
                85                  90                  95

Ala Thr Asn Ile Gly Ser Asp Ala Cys Pro Tyr Gly Ser Gly Ala His
            100                 105                 110

Pro Tyr Leu Thr Leu Gly Thr Ala Thr Ile Asn Arg Leu Glu Leu Arg
        115                 120                 125

Ala Pro Ala Arg Thr Val Leu Gln Ser Asp Glu Arg Gly Leu Pro Ile
    130                 135                 140

Gly Ala Gln Ala Val Glu Gly Thr Glu Tyr Asp Phe Arg Lys Leu Arg
145                 150                 155                 160

Arg Ile Asp Ser Thr Val Leu Asp His Ala Phe Thr Asp Leu Glu Arg
                165                 170                 175

Asp His Asp Gly Leu Ala Arg Val Glu Leu Arg Asp Pro Asp Ser Lys
            180                 185                 190

Thr Gln Val Ser Leu Trp Val Asp Gln Ser Tyr Ser His Leu Met Leu
        195                 200                 205

Phe Thr Gly Asp Pro Gly Leu Thr His
210                 215

<210> 10
<211> 631
<212> DNA
<213> Unknown Organism

<220>
<223> Description of Unknown Organism: nucleotide
      sequence of a clone homologous to β-glucosidase

<400> 10
gaggaaacct acggcgaaga tccgtttctc gcgtcgcgaa tgggcgttgc tgcaatcgaa 60
ggtctgcagg gtgacacatt tctcattggc cgccatcacg tcgtggccac agccaagcac 120
ttcgccgtgc atgggcagcc ggagggtggc actaataccg cgcctgggaa ttattccgaa 180
cgcattattc gcgagaactt tcttgtgccg tttcaggctg cggtagagga agccaaagtc 240
gggagtgtga tggcgtcgta caaggagatc gatggcatcc cgtcgcatgt gaatccctgg 300
ctgcttgatc gggtgctgcg gcaggagtgg ggcttccgag gttatgtcac ctccgacgga 360
gatggacttc agatgctgat ccagacgcat cacgtggccg gaagcaaact tgatgccgcg 420
aggcaggcta ttgcagccgg cgttgactat gacctctccg acgggtcggt gtaccggacg 480
ctgatcggcc aggtcaaaca gggcagcgtt cccgagagcg atgtagaccg cgccgctgcg 540
cgggtgctgg ccactaagtt ccggctggga ttgttcgaca acccgtatgt ggatccgact 600
atgccgacaa accacgaaca gcgcggaaca t                                631

<210> 11

<211> 210

<212> PRT

<213> Unknown Organism

<220>

<223> Description of Unknown Organism: amino acid
      sequence of a clone homologous to β-glucosidase

<400> 11

Glu Glu Thr Tyr Gly Glu Asp Pro Phe Leu Ala Ser Arg Met Gly Val
 1               5                   10                  15

Ala Ala Ile Glu Gly Leu Gln Gly Asp Thr Phe Leu Ile Gly Arg His
            20                  25                  30

His Val Val Ala Thr Ala Lys His Phe Ala Val His Gly Gln Pro Glu
        35                  40                  45

Gly Gly Thr Asn Thr Ala Pro Gly Asn Tyr Ser Glu Arg Ile Ile Arg
    50                  55                  60

Glu Asn Phe Leu Val Pro Phe Gln Ala Ala Val Glu Glu Ala Lys Val
65                  70                  75                  80

Gly Ser Val Met Ala Ser Tyr Lys Glu Ile Asp Gly Ile Pro Ser His
                85                  90                  95

Val Asn Pro Trp Leu Leu Asp Arg Val Leu Arg Gln Glu Trp Gly Phe
            100                 105                 110

Arg Gly Tyr Val Thr Ser Asp Gly Asp Gly Leu Gln Met Leu Ile Gln
        115                 120                 125

Thr His His Val Ala Gly Ser Lys Leu Asp Ala Ala Arg Gln Ala Ile
    130                 135                 140

Ala Ala Gly Val Asp Tyr Asp Leu Ser Asp Gly Ser Val Tyr Arg Thr
145                 150                 155                 160

Leu Ile Gly Gln Val Lys Gln Gly Ser Val Pro Glu Ser Asp Val Asp
                165                 170                 175

Arg Ala Ala Ala Arg Val Leu Ala Thr Lys Phe Arg Leu Gly Leu Phe
            180                 185                 190

Asp Asn Pro Tyr Val Asp Pro Thr Met Pro Thr Asn His Glu Gln Arg
        195                 200                 205

Gly Thr
    210

<210> 12
<211> 258
<212> DNA
<213> Unknown Organism

<220>
<223> Description of Unknown Organism: nucleotide

sequence of a clone homologous to glucoamylase

<400> 12
tggaaaccga accgtcaagt tcgcagcgtc aagcgaggcc acactcttcg aattcaggtt 60
cccgccgcct tccgtctgca ttggtcggac gacgggtggg gatccgtgaa gaacacgcca 120
tcgtccgccg cgatctcggg gatcaacttc gtcgacatcc caatcgctgc tgagcagcag 180
gcgccgattc aattcacgtt tttctggacg gcgactggcc tctgggaagg acgagactac 240
gccgtgagac ccgaatga 258

<210> 13
<211> 85
<212> PRT
<213> Unknown Organism

<220>
<223> Description of Unknown Organism: amino acid
sequence of a clone homologous to glucoamylase

<400> 13
Trp Lys Pro Asn Arg Gln Val Arg Ser Val Lys Arg Gly His Thr Leu
1               5                   10                  15

Arg Ile Gln Val Pro Ala Ala Phe Arg Leu His Trp Ser Asp Asp Gly
            20                  25                  30

Trp Gly Ser Val Lys Asn Thr Pro Ser Ser Ala Ala Ile Ser Gly Ile
        35                  40                  45

Asn Phe Val Asp Ile Pro Ile Ala Ala Glu Gln Gln Ala Pro Ile Gln

50                  55                  60

Phe Thr Phe Phe Trp Thr Ala Thr Gly Leu Trp Glu Gly Arg Asp Tyr
65                  70                  75                  80

Ala Val Arg Pro Glu
                85

<210> 14
<211> 545
<212> DNA
<213> Unknown Organism

<220>
<223> Description of Unknown Organism: nucleotide
      sequence of a clone homologous to glucose dehydratase

<400> 14
cgggaacgtg acgccatccg gcgcctcttt ttcgatgccg atcctcaagt cgtcgtgcac 60
ctggccgccg tcgtgggtgg gatcggcgcg aatcgtctca atccggggcg ctacttctac 120
gaaaatgcga tcatggggct gcagttgatg gaggaagcgc ggctcaatca ggtcgagaaa 180
ttcgtggcgc tcgggacgat ctgttcttat ccgaagttca cggaagttcc ctttcgcgaa 240
gaggacttct ggaacggcta tcccgaagag accaacgcgc cctatggttt ggcgaagaag 300
atgttgctgg ttcaggccca agcttatcgc cagcaatacg gtctgaatgc gatcactctg 360
ttgccggtca atctctacgg tccgcacgac aatttcgatc ctgagtccag tcacgtgatt 420
ccggccctga tccgcaaggc ggtcgaggcg cgcgatctgc ggaaggatca catcgaagtc 480
tgggggacgg ggtcgacgcg gccgcgaatt cggatcatgt ctcctcagcg tttaaacctg 540
cagga                                                             545

<210> 15

<211> 181

<212> PRT

<213> Unknown Organism

<220>

<223> Description of Unknown Organism: amino acid
      sequence of a clone homologous to glucose dehydratase

<400> 15

Arg Glu Arg Asp Ala Ile Arg Arg Leu Phe Phe Asp Ala Asp Pro Gln
  1               5                  10                  15

Val Val Val His Leu Ala Ala Val Val Gly Gly Ile Gly Ala Asn Arg
             20                  25                  30

Leu Asn Pro Gly Arg Tyr Phe Tyr Glu Asn Ala Ile Met Gly Leu Gln
         35                  40                  45

Leu Met Glu Glu Ala Arg Leu Asn Gln Val Glu Lys Phe Val Ala Leu
     50                  55                  60

Gly Thr Ile Cys Ser Tyr Pro Lys Phe Thr Glu Val Pro Phe Arg Glu
 65                  70                  75                  80

Glu Asp Phe Trp Asn Gly Tyr Pro Glu Glu Thr Asn Ala Pro Tyr Gly
                 85                  90                  95

Leu Ala Lys Lys Met Leu Leu Val Gln Ala Gln Ala Tyr Arg Gln Gln

            100                 105                 110

Tyr Gly Leu Asn Ala Ile Thr Leu Leu Pro Val Asn Leu Tyr Gly Pro

        115                 120                 125

His Asp Asn Phe Asp Pro Glu Ser Ser His Val Ile Pro Ala Leu Ile

    130                 135                 140

Arg Lys Ala Val Glu Ala Arg Asp Leu Arg Lys Asp His Ile Glu Val

145                 150                 155                 160

Trp Gly Thr Gly Ser Thr Arg Pro Arg Ile Arg Ile Met Ser Pro Gln

                165                 170                 175

Arg Leu Asn Leu Gln

            180